(19) Europäisches Patentamt
European Patent Office
Office européen des brevets

(11) EP 1 558 722 B1

(12) **EUROPEAN PATENT SPECIFICATION**

(45) Date of publication and mention of the grant of the patent:
**23.04.2008 Bulletin 2008/17**

(21) Application number: **03775308.4**

(22) Date of filing: **06.11.2003**

(51) Int Cl.:
***C12N 1/16*** *(2006.01)* ***C12N 15/81*** *(2006.01)*
***C07K 14/39*** *(2006.01)*

(86) International application number:
**PCT/EP2003/012377**

(87) International publication number:
**WO 2004/042036 (21.05.2004 Gazette 2004/21)**

(54) **PROCESS FOR EXPRESSION AND SECRETION OF PROTEINS BY THE NON-CONVENTIONAL YEAST ZYGOSACCHAROMYCES BAILII**

VERFAHREN ZUR EXPRESSION UND SEKRETION VON PROTEINEN MITTELS DES NICHT-KONVENTIONELLEN HEFE ZYGOSACCHAROMYCES BAILII

METHODE POUR L'EXPRESSION ET LA SECRETION DE PROTEINES PAR LA LEVURE NON CLASSIQUE ZYGOSACCHAROMYCES BAILII

(84) Designated Contracting States:
**AT BE BG CH CY CZ DE DK EE ES FI FR GB GR HU IE IT LI LU MC NL PT RO SE SI SK TR**

(30) Priority: **07.11.2002 DE 10252245**

(43) Date of publication of application:
**03.08.2005 Bulletin 2005/31**

(73) Proprietor: **Porro, Danilo**
**22036 Erba (IT)**

(72) Inventors:
- **PORRO, Danilo**
  **I-22036 Erba (IT)**
- **BRANDUARDI, Paola**
  **I-20133 Mailand (IT)**
- **VALLI, Minoska**
  **I-24050 Calcinate (IT)**
- **ALBERGHINA, Lilia**
  **I-20124 Mailand (IT)**

(74) Representative: **Luderschmidt, Schüler & Partner Patentanwälte**
**John-F.-Kennedy-Strasse 4**
**65189 Wiesbaden (DE)**

(56) References cited:
**EP-A- 0 095 986** **WO-A-00/41477**
**WO-A-98/20035**

- **PATENT ABSTRACTS OF JAPAN vol. 0123, no. 58 (C-531), 26 September 1988 (1988-09-26) & JP 63 112986 A (SUMITOMO CHEM CO LTD), 18 May 1988 (1988-05-18)**
- **SCHMITT M J ET AL: "Killer toxin-secreting double-stranded RNA mycoviruses in the yeasts Hanseniaspora uvarum and Zygosaccharomyces bailii." JOURNAL OF VIROLOGY, vol. 68, no. 3, March 1994 (1994-03), pages 1765-1772,**
- **WEILER F ET AL: "The Zygosaccharomyces bailii antifungal virus toxin zygocin: cloning and expression in a heterologous fungal host." MOLECULAR MICROBIOLOGY, vol. 46, no. 4, November 2002 (2002-11), pages 1095-1105,**
- **RADLER F ET AL: "Investigation of a killer strain of Zygosaccharomyces bailii" JOURNAL OF GENERAL MICROBIOLOGY, vol. 139, 1993, pages 495-500,**
- **WEILER F ET AL: "Zygocin, a secreted antifungal toxin of the yeast Zygosaccharomyces bailii, and its effect on sensitive fungal cells" FEMS YEAST RESEARCH, vol. 3, 2003, pages 69-76,**
- **UTATSU I ET AL: "Yeast plasmids resembling 2um DNA: regional similarities and diversities at the molecular level" JOURNAL OF BACTERIOLOGY, vol. 169, no. 12, December 1987 (1987-12), pages 5537-5545,**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 558 722 B1

SEQUENCE LISTING

<110> Porro, Danilo

<120> Process for expression and secretion of proteins by the non-conventional yeast zygosaccharomyces bailii

<130> p 779wo

<150> DE 10252245.6
<151> 2003-11-07

<160> 95

<170> PatentIn version 3.1

<210> 1
<211> 57
<212> DNA
<213> Saccharomyces cerevisiae

<220>
<221> sig_peptide
<222> (1)..(57)
<223>

<400> 1
cgacagtaaa ttttgccgaa tttaatagct tctactgaaa aacagtggac catgtga 57

<210> 2
<211> 19
<212> PRT
<213> Saccharomyces cerevisiae

<220>
<221> SIGNAL
<222> (1)..(19)
<223>

<400> 2

Met Arg Phe Pro Ser Ile Phe Thr Ala Val Leu Phe Ala Ala Ser Ser
1               5                   10                  15

Ala Leu Ala

<210> 3
<211> 255
<212> DNA
<213> saccharomyces cerevisiae

<220>
<221> sig_peptide
<222> (1)..(255)
<223>

<400> 3

cgacagtaaa ttttgccgaa tttaatagct tctactgaaa aacagtggac catgtgaaaa 60

gatgcatctc atttatcaaa cacataatat tcaagtgagc cttacttcaa ttgtattgaa 120

gtgcaagaaa accaaaaagc aacaacaggt tttggataag tacatatata agagggcctt 180

ttgttcccat caaaaatgtt actgttctta cgattcattt acgattcaag aatagttcaa 240

acaagaagat tacaa 255

<210> 4
<211> 85
<212> PRT
<213> saccharomyces cerevisiae

<220>
<221> SIGNAL
<222> (1)..(85)
<223>

<220>
<221> PROPEP
<222> (20)..(85)
<223>

<400> 4

Met Arg Phe Pro Ser Ile Phe Thr Ala Val Leu Phe Ala Ala Ser Ser
1 5 10 15

Ala Leu Ala Ala Pro Val Asn Thr Thr Thr Glu Asp Glu Thr Ala Gln
20 25 30

Ile Pro Ala Glu Ala Val Ile Gly Tyr Leu Asp Leu Glu Gly Asp Phe
35 40 45

Asp Val Ala Val Leu Pro Phe Ser Asn Ser Thr Asn Asn Gly Leu Leu
50 55 60

Phe Ile Asn Thr Thr Ile Ala Ser Ile Ala Ala Lys Glu Glu Gly Val
65 70 75 80

Ser Leu Asp Lys Arg
85

<210> 5
<211> 63
<212> DNA
<213> Aspergillus niger

<220>
<221> sig_peptide
<222> (1)..(63)
<223>

<400> 5

atgatggtcg cgtggtggtc tctatttctg tacggccttc aggtcgcggc acctgctttg 60

gct 63

<210> 6
<211> 21
<212> PRT
<213> Aspergillus niger

<220>
<221> SIGNAL
<222> (1)..(21)
<223>

<400> 6

Met Met Val Ala Trp Trp Ser Leu Phe Leu Tyr Gly Leu Gln Val Ala
1 5 10 15

Ala Pro Ala Leu Ala
20

<210> 7
<211> 93
<212> DNA
<213> Bacillus sp.

<220>
<221> sig_peptide
<222> (1)..(93)
<223>

<400> 7

atgcaaaaca cagcgaaaaa ctccatctgg cagagggtgc gccacagcgc cattgcctta 60

tccgctctca gtttatcctt tggcctgcag gcc 93

<210> 8
<211> 31
<212> PRT
<213> Bacillus sp.

<220>
<221> SIGNAL
<222> (1)..(31)
<223>

<400> 8

Met Gln Asn Thr Ala Lys Asn Ser Ile Trp Gln Arg Val Arg His Ser
1 5 10 15

Ala Ile Ala Leu Ser Ala Leu Ser Leu Ser Phe Gly Leu Gln Ala
20 25 30

<210> 9
<211> 63
<212> DNA
<213> Aspergillus oryzae

<220>
<221> sig_peptide
<222> (1)..(63)
<223>

<400> 9

atgcagtcca tcaagcgtac cttgctcctc ctcggagcta tccttcccgc ggtcctcggt 60

gcc 63

<210> 10
<211> 21
<212> PRT

<213> Aspergillus oryzae

<220>
<221> SIGNAL
<222> (1)..(21)
<223>

<400> 10

```
Met Gln Ser Ile Lys Arg Thr Leu Leu Leu Leu Gly Ala Ile Leu Pro
1               5                   10                  15

Ala Val Leu Gly Ala
            20
```

<210> 11
<211> 75
<212> DNA
<213> Bacillus amyloliquefaciens

<220>
<221> sig_peptide
<222> (1)..(75)
<223>

<400> 11

```
atgaaacgag tgttgctaat tcttgtcacc ggattgttta tgagtttgtg tgggatcact      60

tctagtgttt cggct                                                       75
```

<210> 12
<211> 25
<212> PRT
<213> Bacillus amyloliquefaciens

<220>
<221> SIGNAL
<222> (1)..(25)
<223>

<400> 12

```
Met Lys Arg Val Leu Leu Ile Leu Val Thr Gly Leu Phe Met Ser Leu
1               5                   10                  15

Cys Gly Ile Thr Ser Ser Val Ser Ala
            20                  25
```

<210> 13
<211> 51
<212> DNA
<213> Saccharomycopsis fibuligera

<220>
<221> sig-peptide
<222> (1)..(51)
<223>

<400> 13
atgttgatga tagtacagct tttggtcttt gcactaggcc ttgctgttgc t 51

<210> 14
<211> 17
<212> PRT
<213> Saccharomycopsis fibuligera

<220>
<221> SIGNAL
<222> (1)..(17)
<223>

<400> 14

Met Leu Met Ile Val Gln Leu Leu Val Phe Ala Leu Gly Leu Ala Val
1 5 10 15

Ala

<210> 15
<211> 51
<212> DNA
<213> Saccharomycopsis fibuligera

<220>
<221> sig_peptide
<222> (1)..(51)
<223>

<400> 15
atgttgttga ttttggaact cttagtactt attatagggc ttggagttgc t 51

<210> 16
<211> 17
<212> PRT
<213> Saccharomycopsis fibuligera

<220>
<221> SIGNAL
<222> (1)..(17)
<223>

<400> 16

Met Leu Leu Ile Leu Glu Leu Leu Val Leu Ile Ile Gly Leu Gly Val
1               5                   10                  15

Ala

<210> 17
<211> 66
<212> DNA
<213> Hypocrea pecorina

<220>
<221> sig_peptide
<222> (1)..(66)
<223>

<400> 17

atggcgccct cagttacact gccgttgacc acggccatcc tggccattgc ccggctcgtc 60

gccgcc 66

<210> 18
<211> 22
<212> PRT
<213> Hypocrea pecorina

<220>
<221> SIGNAL
<222> (1)..(22)
<223>

<400> 18

Met Ala Pro Ser Val Thr Leu Pro Leu Thr Thr Ala Ile Leu Ala Ile
1               5                   10                  15

Ala Arg Leu Val Ala Ala
            20

<210> 19
<211> 63
<212> DNA
<213> Hypocrea pecorina

<220>
<221> sig_peptide
<222> (1)..(63)
<223>

<400> 19

atgaacaagt ccgtggctcc attgctgctt gcagcgtcca tactatatgg cggcgccgtc 60

gca 63

<210> 20
<211> 21
<212> PRT
<213> Hypocrea pecorina

<220>
<221> SIGNAL
<222> (1)..(21)
<223>

<400> 20

Met Asn Lys Ser Val Ala Pro Leu Leu Leu Ala Ala Ser Ile Leu Tyr
1 5 10 15

Gly Gly Ala Val Ala
20

<210> 21
<211> 48
<212> DNA
<213> Arxula adeninivorans

<220>
<221> sig_peptide
<222> (1)..(48)
<223>

<400> 21
atgcgtcagt ttctagcact tgctgctgct gcttccatag cggtggca 48

<210> 22
<211> 18
<212> PRT
<213> Arxula adeninivorans

<220>
<221> SIGNAL
<222> (1)..(18)
<223>

<400> 22

Met Arg Gln Phe Leu Ala Leu Ala Ala Ala Ala Ser Ile Ala Val Ala
1 5 10 15

Asp Ser

<210> 23
<211> 57

<212> DNA
<213> Homo sapiens

<220>
<221> sig_peptide
<222> (1)..(57)
<223>

<400> 23
atgcagcgac tatgtgtgta tgtgctgatc tttgcactgg ctctggccgc cttctct 57

<210> 24
<211> 19
<212> PRT
<213> Homo sapiens

<220>
<221> SIGNAL
<222> (1)..(19)
<223>

<400> 24

```
Met Gln Arg Leu Cys Val Tyr Val Leu Ile Phe Ala Leu Ala Leu Ala
1               5                   10                  15

Ala Phe Ser
```

<210> 25
<211> 75
<212> DNA
<213> Rhizopus oryzae

<220>
<221> sig_peptide
<222> (1)..(75)
<223>

<400> 25

```
atgcaactgt tcaatttgcc attgaaagtt tcattctttc tcgtcctctc ttacttttct      60
ttgctcgttt ctgct                                                      75
```

<210> 26
<211> 25
<212> PRT
<213> Rhizopus oryzae

<220>
<221> SIGNAL
<222> (1)..(25)
<223>

<400> 26

Met Gln Leu Phe Asn Leu Pro Leu Lys Val Ser Phe Phe Leu Val Leu
1               5                   10                  15

Ser Tyr Phe Ser Leu Leu Val Ser Ala
            20                  25

<210> 27
<211> 48
<212> DNA
<213> Aspergillus niger

<220>
<221> sig-peptide
<222> (1)..(48)
<223>

<400> 27
atgcagactc tccttgtgag ctcgcttgtg gtctccctcg ctgcggcc 48

<210> 28
<211> 16
<212> PRT
<213> Aspergillus niger

<220>
<221> SIGNAL
<222> (1)..(16)
<223>

<400> 28

Met Gln Thr Leu Leu Val Ser Ser Leu Val Val Ser Leu Ala Ala Ala
1               5                   10                  15

<210> 29
<211> 54
<212> DNA
<213> Homo sapiens

<220>
<221> sig_peptide
<222> (1)..(54)
<223>

<400> 29
atgaagtggg taacctttat ttcccttctt tttctcttta gctcggctta ttcc 54

<210> 30
<211> 18
<212> PRT
<213> Homo sapiens

<220>

<221> SIGNAL
<222> (1)..(18)
<223>

<400> 30

Met Lys Trp Val Thr Phe Ile Ser Leu Leu Phe Leu Phe Ser Ser Ala
1 5 10 15

Tyr Ser

<210> 31
<211> 78
<212> DNA
<213> Saccharomyces cerevisiae

<220>
<221> sig_peptide
<222> (1)..(78)
<223>

<400> 31

atgacgaagc caacccaagt attagttaga tccgtcagta tattattttt catcacatta 60

ctacacctag tcgtagcg 78

<210> 32
<211> 26
<212> PRT
<213> saccharomyces cerevisiae

<220>
<221> SIGNAL
<222> (1)..(26)
<223>

<400> 32

Met Thr Lys Pro Thr Gln Val Leu Val Arg Ser Val Ser Ile Leu Phe
1 5 10 15

Phe Ile Thr Leu Leu His Leu Val Val Ala
20 25

<210> 33
<211> 63
<212> DNA
<213> Saccharomyces cerevisiae

<220>
<221> sig_peptide

<222> (1)..(63)
<223>

<400> 33

atgggccact tagcgatcct tttcagtatt atcgctgtat tgaatatagc tacagctgtt 60

gca 63

<210> 34
<211> 21
<212> PRT
<213> Saccharomyces cerevisiae

<220>
<221> SIGNAL
<222> (1)..(21)
<223>

<400> 34

Met Gly His Leu Ala Ile Leu Phe Ser Ile Ile Ala Val Leu Asn Ile
1 5 10 15

Ala Thr Ala Val Ala
20

<210> 35
<211> 48
<212> DNA
<213> Kluyveromyces lactis

<220>
<221> sig_peptide
<222> (1)..(48)
<223>

<400> 35
ataaaatgaa tatattttac atatttttgt ttttgctgtc attcgttc 48

<210> 36
<211> 17
<212> PRT
<213> Kluyveromyces lactis

<220>
<221> SIGNAL
<222> (1)..(17)
<223>

<400> 36

Met Asn Ile Phe Tyr Ile Phe Leu Phe Leu Leu Ser Phe Val Gln Gly
1 5 10 15

Leu

<210> 37
<211> 69
<212> DNA
<213> kluyveromyces lactis

<220>
<221> sig-peptide
<222> (1)..(69)
<223>

<400> 37

ataaaatgaa tatattttac atatttttgt ttttgctgtc attcgttcaa ggtttggagc 60

atactcatc 69

<210> 38
<211> 23
<212> PRT
<213> Kluyveromyces lactis

<220>
<221> SIGNAL
<222> (1)..(23)
<223>

<400> 38

Met Lys Ile Tyr His Ile Phe Ser Val Cys Tyr Leu Ile Thr Leu Cys
1 5 10 15

Ala Ala Ala Ala Thr Thr Ala
20

<210> 39
<211> 54
<212> DNA
<213> Saccharomyces cerevisiae

<220>
<221> sig_peptide
<222> (1)..(54)
<223>

<400> 39
atgtttgctt tctactttct caccgcatgc atcagtttga agggcgtttt tggg 54

<210> 40

<211> 18
<212> PRT
<213> Saccharomyces cerevisiae

<220>
<221> SIGNAL
<222> (1)..(18)
<223>

<400> 40

```
Met Phe Ala Phe Tyr Phe Leu Thr Ala Cys Ile Ser Leu Lys Gly Val
1               5                   10                  15

Phe Gly
```

<210> 41
<211> 54
<212> DNA
<213> Saccharomyces cerevisiae

<220>
<221> sig_peptide
<222> (1)..(54)
<223>

<400> 41
atgtttaagt ctgttgttta ttcggttcta gccgctgctt tagttaatgc aggt 54

<210> 42
<211> 18
<212> PRT
<213> Saccharomyces cerevisiae

<220>
<221> SIGNAL
<222> (1)..(18)
<223>

<400> 42

```
Met Phe Lys Ser Val Val Tyr Ser Val Leu Ala Ala Ala Leu Val Asn
1               5                   10                  15

Ala Gly
```

<210> 43
<211> 51
<212> DNA
<213> saccharomyces cerevisiae

<220>
<221> sig_peptide

<222> (1)..(51)
<223>

<400> 43
atgtttaaat ctgttgttta ttcaatttta gccgcttctt tggccaatgc a 51

<210> 44
<211> 17
<212> PRT
<213> Saccharomyces cerevisiae

<220>
<221> SIGNAL
<222> (1)..(17)
<223>

<400> 44

Met Phe Lys Ser Val Val Tyr Ser Ile Leu Ala Ala Ser Leu Ala Asn
1 5 10 15

Ala

<210> 45
<211> 48
<212> DNA
<213> Kluyveromyces lactis

<220>
<221> sig_peptide
<222> (1)..(48)
<223>

<400> 45
atgctatcta ttctgttgag tttattatca ttatcaggga cccatgcg 48

<210> 46
<211> 16
<212> PRT
<213> kluyveromyces lactis

<220>
<221> SIGNAL
<222> (1)..(16)
<223>

<400> 46

Met Leu Ser Ile Leu Leu Ser Leu Leu Ser Leu Ser Gly Thr His Ala
1 5 10 15

<210> 47
<211> 48
<212> DNA

<213> Kluyveromyces lactis

<220>
<221> sig_peptide
<222> (1)..(48)
<223>

<400> 47
atgctatcta ttctgttggg tttattatca ctatcaggga cccatgcg 48

<210> 48
<211> 16
<212> PRT
<213> Kluyveromyces lactis

<220>
<221> SIGNAL
<222> (1)..(16)
<223>

<400> 48

```
Met Leu Ser Ile Leu Leu Gly Leu Leu Ser Leu Ser Gly Thr His Ala
1               5                   10                  15
```

<210> 49
<211> 54
<212> DNA
<213> Aspergillus niger

<220>
<221> sig-peptide
<222> (1)..(54)
<223>

<400> 49
atgggcgtct ctgctgttct acttcctttg tatctcctgt ctggagtcac ctcc 54

<210> 50
<211> 18
<212> PRT
<213> Aspergillus niger

<220>
<221> SIGNAL
<222> (1)..(18)
<223>

<400> 50

Met Gly Val Ser Ala Val Leu Leu Pro Leu Tyr Leu Leu Ser Gly Val
1 5 10 15

Thr Ser

<210> 51
<211> 57
<212> DNA
<213> saccharomyces cerevisiae

<220>
<221> sig_peptide
<222> (1)..(57)
<223>

<400> 51
atgcttttgc aagctttcct tttccttttg gctggttttg cagccaaaat atctgca 57

<210> 52
<211> 19
<212> PRT
<213> Saccharomyces cerevisiae

<220>
<221> SIGNAL
<222> (1)..(19)
<223>

<400> 52

Met Leu Leu Gln Ala Phe Leu Phe Leu Leu Ala Gly Phe Ala Ala Lys
1 5 10 15

Ile Ser Ala

<210> 53
<211> 63
<212> DNA
<213> Saccharomyces cerevisiae

<220>
<221> sig_peptide
<222> (1)..(63)
<223>

<400> 53

atgcaaagac catttctact cgcttatttg gtcctttcgc ttctatttaa ctcggctttg 60

ggt 63

<210> 54
<211> 21
<212> PRT
<213> Saccharomyces cerevisiae

<220>
<221> SIGNAL
<222> (1)..(21)
<223>

<400> 54

Met Gln Arg Pro Phe Leu Leu Ala Tyr Leu Val Leu Ser Leu Leu Phe
1 5 10 15

Asn Ser Ala Leu Gly
20

<210> 55
<211> 63
<212> DNA
<213> saccharomyces cerevisiae

<220>
<221> sig_peptide
<222> (1)..(63)
<223>

<400> 55

atgcaaagac catttctact cgcttatttg gtcctttcgc ttctatttaa ctcagctttg 60

ggt 63

<210> 56
<211> 21
<212> PRT
<213> saccharomyces cerevisiae

<220>
<221> SIGNAL
<222> (1)..(21)
<223>

<400> 56

Met Gln Arg Pro Phe Leu Leu Ala Tyr Leu Val Leu Ser Leu Leu Phe
1 5 10 15

Asn Ser Ala Leu Gly
20

<210> 57
<211> 96
<212> DNA
<213> Saccharomyces cerevisiae

<220>
<221> sig_peptide
<222> (1)..(96)
<223>

<400> 57

atggtaggcc tcaaaaatcc atatacgcac actatgcaaa gaccatttct actcgcttat 60

ttggtccttt cgcttctatt taactcagct ttgggt 96

<210> 58
<211> 32
<212> PRT
<213> Saccharomyces cerevisiae

<220>
<221> SIGNAL
<222> (1)..(32)
<223>

<400> 58

Met Val Gly Leu Lys Asn Pro Tyr Thr His Thr Met Gln Arg Pro Phe
1 5 10 15

Leu Leu Ala Tyr Leu Val Leu Ser Leu Leu Phe Asn Ser Ala Leu Gly
20 25 30

<210> 59
<211> 63
<212> DNA
<213> Zygosaccharomyces bailii

<220>
<221> sig_peptide
<222> (1)..(63)
<223>

<400> 59

atgaaagcag cccaaatatt aacagcaagt atagtaagct tattgccaat atatactagt 60

gct 63

<210> 60
<211> 21
<212> PRT
<213> Zygosaccharomyces bailii

<220>
<221> SIGNAL
<222> (1)..(21)
<223>

<400> 60

Met Lys Ala Ala Gln Ile Leu Thr Ala Ser Ile Val Ser Leu Leu Pro
1 5 10 15

Ile Tyr Thr Ser Ala
20

<210> 61
<211> 417
<212> DNA
<213> Zygosaccharomyces bailii

<220>
<221> sig_peptide
<222> (1)..(417)
<223>

<400> 61

atgaaagcag cccaaatatt aacagcaagt atagtaagct tattgccaat atatactagt 60

gctagaaaca tattagacag agaatacaca gcaaacgaat taaaaactgc ttttggagat 120

gaagaaattt ttacagattt gacgtatcac attcacgtta acgtcagtgg cgaaattgac 180

tcttactatc ataatttagt caattttgtc gataacgctc tagcaaacaa agatattaat 240

agatatatat acgctatatt tacacagcag acaaactata cagaggatgg gctcattgag 300

tacttaaatc attacgattc agagacttgc aaagatatca ttactcagta taatgttaac 360

gtagacacta gtaactgtat aagcaatact acagatcaag ctagactcca acgtcgc 417

<210> 62
<211> 139
<212> PRT
<213> Zygosaccharomyces bailii

<220>
<221> SIGNAL

<222> (1)..(139)
<223>

<220>
<221> PROPEP
<222> (22)..(139)
<223>

<400> 62

```
Met Lys Ala Ala Gln Ile Leu Thr Ala Ser Ile Val Ser Leu Leu Pro
1               5                   10                  15

Ile Tyr Thr Ser Ala Arg Asn Ile Leu Asp Arg Glu Tyr Thr Ala Asn
            20                  25                  30

Glu Leu Lys Thr Ala Phe Gly Asp Glu Glu Ile Phe Thr Asp Leu Thr
        35                  40                  45

Tyr His Ile His Val Asn Val Ser Gly Glu Ile Asp Ser Tyr Tyr His
    50                  55                  60

Asn Leu Val Asn Phe Val Asp Asn Ala Leu Ala Asn Lys Asp Ile Asn
65                  70                  75                  80

Arg Tyr Ile Tyr Ala Ile Phe Thr Gln Gln Thr Asn Tyr Thr Glu Asp
                85                  90                  95

Gly Leu Ile Glu Tyr Leu Asn His Tyr Asp Ser Glu Thr Cys Lys Asp
            100                 105                 110

Ile Ile Thr Gln Tyr Asn Val Asn Val Asp Thr Ser Asn Cys Ile Ser
        115                 120                 125

Asn Thr Thr Asp Gln Ala Arg Leu Gln Arg Arg
    130                 135
```

<210> 63
<211> 587
<212> DNA
<213> Zygosaccharomyces bailii

<400> 63

ttatagatct aaaaataaat aatataaatt acctcatcca gagtcccccc ccaaatcctt 60
attctaaata ataagctact cctccccccc caggagtatt tttaggggga ggggggacct 120
taactcaagg gggagtagtt ttgaggatca catgggaagt atttaaataa atagtagttc 180
ttttgtttta aaaaggcctc tccaaaagta atacttttag ggtaattact aagtataata 240
tatattataa gtaatagcct ttatagctta atggtaaagc agtaaattga agatttacct 300
atatgtagtt cgattctcat taagggcaat ataaataagc tttttaatgg gccaatagct 360
gaaataagta atattattgt aaatattgag acttgaactc aaatcttatg cacctaaaaa 420
catatatttt aaccaattaa attatattta ctttattatt tacttatata acttctacta 480
attgtaaagt ataaccagct tttttgttaa caacaaaaac cgagagggtt catgttatat 540
ataatttata attgttctta ctttatttat aaaagaataa ccgaatg 587

<210> 64
<211> 435
<212> DNA
<213> Zygosaccharomyces bailii

<400> 64

ttaatttaat ttgtcgctaa ataataatgt tttaaatatt ataaatattt caaccaacca 60
cccccccaa aagggggtgg ttggtggttg gtcgtcacca accacctttg gtgggtggtg 120
ccccctatga gttttcatat tataaatata aaaactttta tggagggacc tataagaaat 180
aattgaggaa taattaataa taagttgccc tccttttttt tctcttctcc ccaccctaaa 240
aatactcctg gggggggag ggagagaatg tatgtagtgg ggagggtgta agttaataat 300
agacttaaat agagttatat aaaataacat aaatatgctt aaaaataata ataataatat 360
taacagatag aagccaaagg gtcaggcgct ttctttggga gaaagagtta gttagttcga 420
atctatccta tctga 435

<210> 65
<211> 299
<212> DNA
<213> Zygosaccharomyces bailii

<400> 65

ttttggggtc gaggtgccgt aaagcactaa atcggaaccc taaagggagc cccgatttta 60
gagcttgacg gggaaagccg gcgaacgtgg cgagaaagga agggaagaaa gcgaaaggag 120
cgggcgctag ggcgctggca agtgtagcgg tcacgctgcg cgtaaccacc acacccgccg 180
cgcttaatgc gccgctacag ggcgcgtcag gtggcacttt tcggggaaat gtgcgcggaa 240
cccctatttg tttatttttc taaatacatt caaatatgta tccgctcatg agacaataa 299

<210> 66
<211> 153
<212> DNA
<213> Zygosaccharomyces bailii

<400> 66

ctccactgta acatttccca ctgtcctttt cccatctttc attttacaat gagcaagttt 60
cagaaaaaaa aatacaaatg ggataagtgc aaaacattcc atgtatctgt agcttccaat 120
gttattcctc tctccagagt caggcttctg tgt 153

<210> 67
<211> 231
<212> DNA
<213> Zygosaccharomyces bailii

<400> 67

aattcaatag atgatgattt aacttcattt aatgagaaga tttcattaga ttcaacaaaa 60
tctggagatt ttgcataaac aactgattta ttattagctt tattttctaa tccattaact 120
aattgatcat acataatata gatgaataag aataatgaaa ctagtgcaat aattgatcca 180
attgatgcta cataatttca accagcaaag gcatcagggt agtcaggaat t 231

<210> 68
<211> 52
<212> DNA
<213> Zygosaccharomyces bailii

<400> 68
ctcgtaaaaa cgagcatgag ctgcgtcagg tcagccgtgg atatcgttgc gg 52

<210> 69
<211> 116
<212> DNA
<213> Zygosaccharomyces bailii

<400> 69

ctatctgcac gtgccaccgg aggtgctgtg ggagcgactg cggcacgatc gccatcggcc 60
gctgctgcag gtgccgcagc cgaggcagcg cattttcgaa ctctacgccc agcgcg 116

<210> 70
<211> 268
<212> DNA
<213> Zygosaccharomyces bailii

<400> 70

ccggcccctt gttgcgggcg gtgccgatca ggcgcagcga gttgatctcg cgcagccggt 60
cctgggcata attgagcaaa tcgtactcgt gcgcggcgat gcgctccttg ccgatcgaat 120
tgacgtaatc gatcgcggcg ccgagcccga tcgcctcgac gatcggcggc gtgccggcct 180
cgaacttgtg cggcgggtcg ccataggtga cccagtcctt ggcaacttca cggatcattt 240
cgccgccgcc gttgaacggc cgcatcgc 268

<210> 71
<211> 869
<212> DNA
<213> Zygosaccharomyces bailii

<400> 71

cacacagaaa cagctatgac catgatacgc caagcttaat acgactcact ataggaaagc 60
tcggtaccac gcatgctgca gacgcgttac gtatcggatc cagaattcgt gatattctat 120
tgggtatgtc ccctgattcg acggcgtaaa ttgcgtgaat cttgtgttgg cgctaatgac 180
cgctttttgg aattatgtgc tatgcctctg ccattggtat caacagctga aatatttgtt 240
gaagatcgaa tatcttctat tgtttctgag ggtatccccg aagctatggc gaaagaaagg 300
atctcttctc gtacttggat cggtacgaga agcaatagac gcacaatgca ttgacgcatc 360
ttgttgatac cgggtaatgt gagtcttctg ggttctgtta ttgagtttaa tatgtcgtcc 420
acctctgttc tcgtatccat tttgcgagta gcccgccata cagcacgtcc aatacaggag 480
aggccattta gcttcaggtg cagagaagac acagcatggt gctcaccttc gagtgtctca 540
atagatgatt gagttgactg ggcttccgtg aaagggcctt tcgagagatc ttcagaaata 600
aaccagggtt gcgcttcatt agtaggtgtt cctggaggac tattgtcgct atctgctgga 660
ctactgctac caagtagtga agggggtatt ctaaggcttt cactctgttc tgacactatt 720
ataacattgc caaggccaat ttgaaaggtt tcgcgtatat gagtaaagag ctcggtgccc 780
ttccagttgg aatcaagccg ttcaagcaga tcgagagcat aatcagagtc cacatttccg 840
cacgcaagag agaactctga gttcattct 869

<210> 72
<211> 1425
<212> DNA
<213> Zygosaccharomyces bailii

<220>
<221> CDS
<222> (1)..(1425)
<223>

<400> 72

```
atg tcc gag ttt agc gag ctt gtc aga att ctc cca tta gac cag gtt      48
Met Ser Glu Phe Ser Glu Leu Val Arg Ile Leu Pro Leu Asp Gln Val
1               5                   10                  15
```

```
gca gaa ata aag cgt att ttg agt cgc ggc gac cct ata cct tta caa      96
Ala Glu Ile Lys Arg Ile Leu Ser Arg Gly Asp Pro Ile Pro Leu Gln
            20                  25                  30

agg tta gct tct cta cta act atg gtg atc cta acg gtc aac atg tca     144
Arg Leu Ala Ser Leu Leu Thr Met Val Ile Leu Thr Val Asn Met Ser
        35                  40                  45

aaa aag agg aag agc tct cca atc aag ctt agc acc ttt act aaa tat     192
Lys Lys Arg Lys Ser Ser Pro Ile Lys Leu Ser Thr Phe Thr Lys Tyr
    50                  55                  60

cgt aga aat gtt gcg aag tca ttg tat tat gat atg tca agc aag aca     240
Arg Arg Asn Val Ala Lys Ser Leu Tyr Tyr Asp Met Ser Ser Lys Thr
65                  70                  75                  80

gta ttc ttc gaa tac cat ctc aaa aat aca caa gat cta cag gag ggc     288
Val Phe Phe Glu Tyr His Leu Lys Asn Thr Gln Asp Leu Gln Glu Gly
                85                  90                  95

ctc gag caa gcc att gcg ccc tac aat ttc gtg gta aag gtg cac aag     336
Leu Glu Gln Ala Ile Ala Pro Tyr Asn Phe Val Val Lys Val His Lys
            100                 105                 110

aag cca att gat tgg cag aaa cag ctc tca agc gtg cat gag agg aaa     384
Lys Pro Ile Asp Trp Gln Lys Gln Leu Ser Ser Val His Glu Arg Lys
        115                 120                 125

gcg ggc cac aga agc att ctc agc aac aat gtt ggc gcc gag atc tct     432
Ala Gly His Arg Ser Ile Leu Ser Asn Asn Val Gly Ala Glu Ile Ser
    130                 135                 140

aaa ctg gct gag acg aaa gat tct act tgg agt ttt atc gag aga aca     480
Lys Leu Ala Glu Thr Lys Asp Ser Thr Trp Ser Phe Ile Glu Arg Thr
145                 150                 155                 160

atg gat ctg ata gaa gcc cgc acc cgc cag ccc acg aca aga gtt gcg     528
Met Asp Leu Ile Glu Ala Arg Thr Arg Gln Pro Thr Thr Arg Val Ala
                165                 170                 175

tat agg ttt ctg ctt caa ctc aca ttc atg aac tgc tgt agg gct aat     576
Tyr Arg Phe Leu Leu Gln Leu Thr Phe Met Asn Cys Cys Arg Ala Asn
            180                 185                 190

gat ttg aaa aac gcc gac ccc agc act ttt caa atc atc gca gat cct     624
Asp Leu Lys Asn Ala Asp Pro Ser Thr Phe Gln Ile Ile Ala Asp Pro
        195                 200                 205

cac ctt ggt cgt ata ttg cgg gcc ttt gtt cca gag aca aag act agc     672
His Leu Gly Arg Ile Leu Arg Ala Phe Val Pro Glu Thr Lys Thr Ser
    210                 215                 220

att gaa agg ttt atc tat ttt ttc cca tgt aag gga cga tgc gat ccg     720
Ile Glu Arg Phe Ile Tyr Phe Phe Pro Cys Lys Gly Arg Cys Asp Pro
225                 230                 235                 240

ctt ttg gct cta gat tcc tat ctc ctg tgg gtt ggc cca gtg ccc aaa     768
Leu Leu Ala Leu Asp Ser Tyr Leu Leu Trp Val Gly Pro Val Pro Lys
                245                 250                 255

act cag act acc gat gaa gag act caa tat gat tac cag ctt ctt caa     816
Thr Gln Thr Thr Asp Glu Glu Thr Gln Tyr Asp Tyr Gln Leu Leu Gln
            260                 265                 270

gat act ctc ttg att tcg tac gac agg ttt atc gcc aaa gaa tca aag     864
Asp Thr Leu Leu Ile Ser Tyr Asp Arg Phe Ile Ala Lys Glu Ser Lys
        275                 280                 285
```

```
gaa aat att ttc aaa ata cct aat ggg ccc aaa gct cat ttg ggg cgg      912
Glu Asn Ile Phe Lys Ile Pro Asn Gly Pro Lys Ala His Leu Gly Arg
    290                 295                 300

cat cta atg gca tca tac ctt gga aac aac agt ctc aag agc gag gcc      960
His Leu Met Ala Ser Tyr Leu Gly Asn Asn Ser Leu Lys Ser Glu Ala
305                 310                 315                 320

aca ctc tac ggc aac tgg tct gtg gaa agg caa gag ggc gtc agc aaa     1008
Thr Leu Tyr Gly Asn Trp Ser Val Glu Arg Gln Glu Gly Val Ser Lys
                325                 330                 335

atg gct gac agc cga tac atg cac acg gtt aaa aaa agt cca cct tca     1056
Met Ala Asp Ser Arg Tyr Met His Thr Val Lys Lys Ser Pro Pro Ser
            340                 345                 350

tat cta ttt gca ttt tta tcc ggc tac tac aaa aag tcc aac caa ggc     1104
Tyr Leu Phe Ala Phe Leu Ser Gly Tyr Tyr Lys Lys Ser Asn Gln Gly
        355                 360                 365

gag tac gtg ctg gct gaa aca ctg tat aat ccc ctg gat tac gac aaa     1152
Glu Tyr Val Leu Ala Glu Thr Leu Tyr Asn Pro Leu Asp Tyr Asp Lys
    370                 375                 380

aca ctt cca ata aca acg aac gag aaa ttg atc tgt cgg cgg tac ggg     1200
Thr Leu Pro Ile Thr Thr Asn Glu Lys Leu Ile Cys Arg Arg Tyr Gly
385                 390                 395                 400

aaa aat gcg aaa gtg ata cca aaa gac gca ctg ctg tat ctc tac acg     1248
Lys Asn Ala Lys Val Ile Pro Lys Asp Ala Leu Leu Tyr Leu Tyr Thr
                405                 410                 415

tat gcg cag cag aag cga aaa caa ttg gcc gat ccc aat gag caa aat     1296
Tyr Ala Gln Gln Lys Arg Lys Gln Leu Ala Asp Pro Asn Glu Gln Asn
            420                 425                 430

agg cta ttc agt agt gaa tca cca gcg cat ccc ttc tta act cct caa     1344
Arg Leu Phe Ser Ser Glu Ser Pro Ala His Pro Phe Leu Thr Pro Gln
        435                 440                 445

tcg aca ggc tca tcg aca ccc ttg acc tgg act gct cca aag aca ctc     1392
Ser Thr Gly Ser Ser Thr Pro Leu Thr Trp Thr Ala Pro Lys Thr Leu
    450                 455                 460

tcc act ggt cta atg aca cct gga gaa gag tag                         1425
Ser Thr Gly Leu Met Thr Pro Gly Glu Glu
465                 470
```

<210> 73
<211> 474
<212> PRT
<213> Zygosaccharomyces bailii

<400> 73

```
Met Ser Glu Phe Ser Glu Leu Val Arg Ile Leu Pro Leu Asp Gln Val
1               5                   10                  15

Ala Glu Ile Lys Arg Ile Leu Ser Arg Gly Asp Pro Ile Pro Leu Gln
            20                  25                  30
```

```
Arg Leu Ala Ser Leu Leu Thr Met Val Ile Leu Thr Val Asn Met Ser
        35                  40                  45

Lys Lys Arg Lys Ser Ser Pro Ile Lys Leu Ser Thr Phe Thr Lys Tyr
    50                  55                  60

Arg Arg Asn Val Ala Lys Ser Leu Tyr Tyr Asp Met Ser Ser Lys Thr
65                  70                  75                  80

Val Phe Phe Glu Tyr His Leu Lys Asn Thr Gln Asp Leu Gln Glu Gly
                85                  90                  95

Leu Glu Gln Ala Ile Ala Pro Tyr Asn Phe Val Val Lys Val His Lys
            100                 105                 110

Lys Pro Ile Asp Trp Gln Lys Gln Leu Ser Ser Val His Glu Arg Lys
        115                 120                 125

Ala Gly His Arg Ser Ile Leu Ser Asn Asn Val Gly Ala Glu Ile Ser
    130                 135                 140

Lys Leu Ala Glu Thr Lys Asp Ser Thr Trp Ser Phe Ile Glu Arg Thr
145                 150                 155                 160

Met Asp Leu Ile Glu Ala Arg Thr Arg Gln Pro Thr Thr Arg Val Ala
                165                 170                 175

Tyr Arg Phe Leu Leu Gln Leu Thr Phe Met Asn Cys Cys Arg Ala Asn
            180                 185                 190

Asp Leu Lys Asn Ala Asp Pro Ser Thr Phe Gln Ile Ile Ala Asp Pro
        195                 200                 205

His Leu Gly Arg Ile Leu Arg Ala Phe Val Pro Glu Thr Lys Thr Ser
    210                 215                 220

Ile Glu Arg Phe Ile Tyr Phe Phe Pro Cys Lys Gly Arg Cys Asp Pro
225                 230                 235                 240

Leu Leu Ala Leu Asp Ser Tyr Leu Leu Trp Val Gly Pro Val Pro Lys
                245                 250                 255

Thr Gln Thr Thr Asp Glu Glu Thr Gln Tyr Asp Tyr Gln Leu Leu Gln
            260                 265                 270

Asp Thr Leu Leu Ile Ser Tyr Asp Arg Phe Ile Ala Lys Glu Ser Lys
        275                 280                 285

Glu Asn Ile Phe Lys Ile Pro Asn Gly Pro Lys Ala His Leu Gly Arg
    290                 295                 300
```

```
His Leu Met Ala Ser Tyr Leu Gly Asn Asn Ser Leu Lys Ser Glu Ala
305                 310                 315                 320

Thr Leu Tyr Gly Asn Trp Ser Val Glu Arg Gln Glu Gly Val Ser Lys
                325                 330                 335

Met Ala Asp Ser Arg Tyr Met His Thr Val Lys Lys Ser Pro Pro Ser
            340                 345                 350

Tyr Leu Phe Ala Phe Leu Ser Gly Tyr Tyr Lys Lys Ser Asn Gln Gly
        355                 360                 365

Glu Tyr Val Leu Ala Glu Thr Leu Tyr Asn Pro Leu Asp Tyr Asp Lys
    370                 375                 380

Thr Leu Pro Ile Thr Thr Asn Glu Lys Leu Ile Cys Arg Arg Tyr Gly
385                 390                 395                 400

Lys Asn Ala Lys Val Ile Pro Lys Asp Ala Leu Leu Tyr Leu Tyr Thr
                405                 410                 415

Tyr Ala Gln Gln Lys Arg Lys Gln Leu Ala Asp Pro Asn Glu Gln Asn
            420                 425                 430

Arg Leu Phe Ser Ser Glu Ser Pro Ala His Pro Phe Leu Thr Pro Gln
        435                 440                 445

Ser Thr Gly Ser Ser Thr Pro Leu Thr Trp Thr Ala Pro Lys Thr Leu
    450                 455                 460

Ser Thr Gly Leu Met Thr Pro Gly Glu Glu
465                 470
```

<210> 74
<211> 1074
<212> DNA
<213> Zygosaccharomyces bailii

<220>
<221> CDS
<222> (1)..(1074)
<223>

<400> 74

```
atg ttc tcc agg gaa gag gtt agg gcc tcc aaa ccc act aaa gag atg      48
```

```
Met Phe Ser Arg Glu Glu Val Arg Ala Ser Arg Pro Thr Lys Glu Met
1               5                   10                  15

aag atg atc ttt gat gtg ctt atg aca ttt cct tac ttc gcg gta cat      96
Lys Met Ile Phe Asp Val Leu Met Thr Phe Pro Tyr Phe Ala Val His
            20                  25                  30

gtt cct tcc aag aat ata ctt atc aca cca aaa ggc aca gtt gag ata     144
Val Pro Ser Lys Asn Ile Leu Ile Thr Pro Lys Gly Thr Val Glu Ile
        35                  40                  45

cct gaa aac tat caa aat tat ccc ata ttg gcc atc ttc tac gtc aaa     192
Pro Glu Asn Tyr Gln Asn Tyr Pro Ile Leu Ala Ile Phe Tyr Val Lys
    50                  55                  60

tat tta atg aag aaa aat ccg tac gat ctt ctt cca agc acc gtg aac     240
Tyr Leu Met Lys Lys Asn Pro Tyr Asp Leu Leu Pro Ser Thr Val Asn
65                  70                  75                  80

tgg ccg gaa ccc tat gta gtg gtg aat acc atc act aag cgt ttc cag     288
Trp Pro Glu Pro Tyr Val Val Val Asn Thr Ile Thr Lys Arg Phe Gln
                85                  90                  95

gac cat aaa cta ttt gca aac aaa aat gct gat gtc tac gtt gaa aga     336
Asp His Lys Leu Phe Ala Asn Lys Asn Ala Asp Val Tyr Val Glu Arg
            100                 105                 110

ctt caa aat gca att gcc tcg ggt att aag att cct gag tct aag aag     384
Leu Gln Asn Ala Ile Ala Ser Gly Ile Lys Ile Pro Glu Ser Lys Lys
        115                 120                 125

aat gaa cga tta ggg cag cca aaa aag acg aaa aat gtt aca aaa gag     432
Asn Glu Arg Leu Gly Gln Pro Lys Lys Thr Lys Asn Val Thr Lys Glu
    130                 135                 140

att gag gag acc ttt att gat gcc act aat gcg aga aaa gaa ttg gat     480
Ile Glu Glu Thr Phe Ile Asp Ala Thr Asn Ala Arg Lys Glu Leu Asp
145                 150                 155                 160

gag tac ttc aga aaa ctt cag gat ggt aca tta acc gga gat ttg gag     528
Glu Tyr Phe Arg Lys Leu Gln Asp Gly Thr Leu Thr Gly Asp Leu Glu
                165                 170                 175

ggt ggc ttg tgc aag gtc aaa acg ctc ata tcg tgt aaa gct ttg ttc     576
Gly Gly Leu Cys Lys Val Lys Thr Leu Ile Ser Cys Lys Ala Leu Phe
            180                 185                 190

gga gga cac acc caa gaa ctc cag ttt atg gcc acc aat gtt cgt aaa     624
Gly Gly His Thr Gln Glu Leu Gln Phe Met Ala Thr Asn Val Arg Lys
        195                 200                 205

gtc tgg ata ggg gag ata gtg tgc ggc atg gtt tcc aat aaa aat gca     672
Val Trp Ile Gly Glu Ile Val Cys Gly Met Val Ser Asn Lys Asn Ala
    210                 215                 220

att gac gat aat gat ctc gag gaa gaa gag cgt aat gca tcg ggc gaa     720
Ile Asp Asp Asn Asp Leu Glu Glu Glu Glu Arg Asn Ala Ser Gly Glu
225                 230                 235                 240

caa act acg aca gcc cga gag gaa tca gag gct ctg gat acc aca tcc     768
Gln Thr Thr Thr Ala Arg Glu Glu Ser Glu Ala Leu Asp Thr Thr Ser
                245                 250                 255

aat ggt ttg gac gct ctg aat act caa att aat gcc ata gaa acg gag     816
Asn Gly Leu Asp Ala Leu Asn Thr Gln Ile Asn Ala Ile Glu Thr Glu
            260                 265                 270

gaa tca ttt tgg gaa gct atc agg gcg ctc cat aat gag cta cgc acc     864
```

```
Glu Ser Phe Trp Glu Ala Ile Arg Ala Leu His Asn Glu Leu Arg Thr
    275                     280                 285

tct cca aca cag tta gaa gag tgc agg aaa gcg gca gtt ttt tta ctg      912
Ser Pro Thr Gln Leu Glu Glu Cys Arg Lys Ala Ala Val Phe Leu Leu
    290                 295                 300

ggc cat aaa aaa ata ctc caa aca ttt aca aag caa aag gat act gcc      960
Gly His Lys Lys Ile Leu Gln Thr Phe Thr Lys Gln Lys Asp Thr Ala
305                 310                 315                 320

cgc gct ctt ttt tat ata aat ctc aaa gag tgt ctg gga acc agc tgg     1008
Arg Ala Leu Phe Tyr Ile Asn Leu Lys Glu Cys Leu Gly Thr Ser Trp
                325                 330                 335

aat tta gaa tat aca gag gca tca gat gca aga aaa atg gca att aaa     1056
Asn Leu Glu Tyr Thr Glu Ala Ser Asp Ala Arg Lys Met Ala Ile Lys
            340                 345                 350

ggt gag ctt caa aat taa                                             1074
Gly Glu Leu Gln Asn
        355
```

<210> 75
<211> 357
<212> PRT
<213> Zygosaccharomyces bailii

<400> 75

```
Met Phe Ser Arg Glu Glu Val Arg Ala Ser Arg Pro Thr Lys Glu Met
1               5                   10                  15

Lys Met Ile Phe Asp Val Leu Met Thr Phe Pro Tyr Phe Ala Val His
            20                  25                  30

Val Pro Ser Lys Asn Ile Leu Ile Thr Pro Lys Gly Thr Val Glu Ile
        35                  40                  45

Pro Glu Asn Tyr Gln Asn Tyr Pro Ile Leu Ala Ile Phe Tyr Val Lys
    50                  55                  60

Tyr Leu Met Lys Lys Asn Pro Tyr Asp Leu Leu Pro Ser Thr Val Asn
65                  70                  75                  80

Trp Pro Glu Pro Tyr Val Val Val Asn Thr Ile Thr Lys Arg Phe Gln
                85                  90                  95

Asp His Lys Leu Phe Ala Asn Lys Asn Ala Asp Val Tyr Val Glu Arg
            100                 105                 110

Leu Gln Asn Ala Ile Ala Ser Gly Ile Lys Ile Pro Glu Ser Lys Lys
        115                 120                 125
```

```
Asn Glu Arg Leu Gly Gln Pro Lys Lys Thr Lys Asn Val Thr Lys Glu
    130                 135                 140

Ile Glu Glu Thr Phe Ile Asp Ala Thr Asn Ala Arg Lys Glu Leu Asp
145                 150                 155                 160

Glu Tyr Phe Arg Lys Leu Gln Asp Gly Thr Leu Thr Gly Asp Leu Glu
                165                 170                 175

Gly Gly Leu Cys Lys Val Lys Thr Leu Ile Ser Cys Lys Ala Leu Phe
            180                 185                 190

Gly Gly His Thr Gln Glu Leu Gln Phe Met Ala Thr Asn Val Arg Lys
        195                 200                 205

Val Trp Ile Gly Glu Ile Val Cys Gly Met Val Ser Asn Lys Asn Ala
    210                 215                 220

Ile Asp Asp Asn Asp Leu Glu Glu Glu Glu Arg Asn Ala Ser Gly Glu
225                 230                 235                 240

Gln Thr Thr Thr Ala Arg Glu Glu Ser Glu Ala Leu Asp Thr Thr Ser
                245                 250                 255

Asn Gly Leu Asp Ala Leu Asn Thr Gln Ile Asn Ala Ile Glu Thr Glu
            260                 265                 270

Glu Ser Phe Trp Glu Ala Ile Arg Ala Leu His Asn Glu Leu Arg Thr
        275                 280                 285

Ser Pro Thr Gln Leu Glu Glu Cys Arg Lys Ala Ala Val Phe Leu Leu
    290                 295                 300

Gly His Lys Lys Ile Leu Gln Thr Phe Thr Lys Gln Lys Asp Thr Ala
305                 310                 315                 320

Arg Ala Leu Phe Tyr Ile Asn Leu Lys Glu Cys Leu Gly Thr Ser Trp
                325                 330                 335

Asn Leu Glu Tyr Thr Glu Ala Ser Asp Ala Arg Lys Met Ala Ile Lys
            340                 345                 350

Gly Glu Leu Gln Asn
        355
```

<210> 76
<211> 750
<212> DNA
<213> Zygosaccharomyces bailii

<220>
<221> CDS

<222> (1)..(750)
<223>

<400> 76

```
atg aac tca gag ttc tct ctt gcg tac gga aat gtg gac tct gat tat       48
Met Asn Ser Glu Phe Ser Leu Ala Tyr Gly Asn Val Asp Ser Asp Tyr
1               5                   10                  15

gct ctc gat ctg ctt gaa cgg ctt gat tcc aac tgg aag ggc acc gag       96
Ala Leu Asp Leu Leu Glu Arg Leu Asp Ser Asn Trp Lys Gly Thr Glu
            20                  25                  30

ctc ttt act cat ata cgc gaa acc ttt caa att ggc ctt ggc aat gtt      144
Leu Phe Thr His Ile Arg Glu Thr Phe Gln Ile Gly Leu Gly Asn Val
        35                  40                  45

atc ata gtg tca gaa cag agt gaa agc ctt aga ata ccc cct tca cta      192
Ile Ile Val Ser Glu Gln Ser Glu Ser Leu Arg Ile Pro Pro Ser Leu
    50                  55                  60

ctt ggt agc agt agt cca gca gat agc gac aat agt cct cca gga aca      240
Leu Gly Ser Ser Ser Pro Ala Asp Ser Asp Asn Ser Pro Pro Gly Thr
65                  70                  75                  80

cct act aat gaa gcg caa ccc tgg ttt att tct gaa gat ctc tcg aaa      288
Pro Thr Asn Glu Ala Gln Pro Trp Phe Ile Ser Glu Asp Leu Ser Lys
                85                  90                  95

ggc cct ttc acg gaa gcc cag tca act caa tca tct att gag aca ctc      336
Gly Pro Phe Thr Glu Ala Gln Ser Thr Gln Ser Ser Ile Glu Thr Leu
            100                 105                 110

gaa ggt gag cac cat gct gtg tct tct ctg cac ctg aag cta aat ggc      384
Glu Gly Glu His His Ala Val Ser Ser Leu His Leu Lys Leu Asn Gly
        115                 120                 125

ctc tcc tgt att gga cgt gct gta tgg cgg gct act cgc aaa atg gat      432
Leu Ser Cys Ile Gly Arg Ala Val Trp Arg Ala Thr Arg Lys Met Asp
    130                 135                 140

acg aga aca gag gtg gac gac ata tta aac tca ata aca gaa ccc aga      480
Thr Arg Thr Glu Val Asp Asp Ile Leu Asn Ser Ile Thr Glu Pro Arg
145                 150                 155                 160

aga ctc aca tta ccc ggt atc aac aag atg cgt caa tgc att gtg cgt      528
Arg Leu Thr Leu Pro Gly Ile Asn Lys Met Arg Gln Cys Ile Val Arg
                165                 170                 175

cta ttg ctt ctc gta ccg atc caa gta cga gaa gag atc ctt tct ttc      576
Leu Leu Leu Leu Val Pro Ile Gln Val Arg Glu Glu Ile Leu Ser Phe
            180                 185                 190

gcc ata gct tcg ggg ata ccc tca gaa aca ata gaa gat att cga tct      624
Ala Ile Ala Ser Gly Ile Pro Ser Glu Thr Ile Glu Asp Ile Arg Ser
        195                 200                 205

tca aca aat att tca gct gtt gat acc aat ggc aga ggc ata gca cat      672
Ser Thr Asn Ile Ser Ala Val Asp Thr Asn Gly Arg Gly Ile Ala His
    210                 215                 220
```

```
aat tcc aaa aag cgg tca tta gcg cca aca caa gat tca cgc aat tta       720
Asn Ser Lys Lys Arg Ser Leu Ala Pro Thr Gln Asp Ser Arg Asn Leu
225                 230                 235                 240

cgc cgt cga atc agg gga cat acc caa tag                               750
Arg Arg Arg Ile Arg Gly His Thr Gln
                245
```

<210> 77
<211> 249
<212> PRT
<213> Zygosaccharomyces bailii

<400> 77

```
Met Asn Ser Glu Phe Ser Leu Ala Tyr Gly Asn Val Asp Ser Asp Tyr
1               5                   10                  15

Ala Leu Asp Leu Leu Glu Arg Leu Asp Ser Asn Trp Lys Gly Thr Glu
            20                  25                  30

Leu Phe Thr His Ile Arg Glu Thr Phe Gln Ile Gly Leu Gly Asn Val
        35                  40                  45

Ile Ile Val Ser Glu Gln Ser Glu Ser Leu Arg Ile Pro Pro Ser Leu
    50                  55                  60

Leu Gly Ser Ser Ser Pro Ala Asp Ser Asp Asn Ser Pro Pro Gly Thr
65                  70                  75                  80

Pro Thr Asn Glu Ala Gln Pro Trp Phe Ile Ser Glu Asp Leu Ser Lys
                85                  90                  95

Gly Pro Phe Thr Glu Ala Gln Ser Thr Gln Ser Ser Ile Glu Thr Leu
            100                 105                 110

Glu Gly Glu His His Ala Val Ser Ser Leu His Leu Lys Leu Asn Gly
        115                 120                 125

Leu Ser Cys Ile Gly Arg Ala Val Trp Arg Ala Thr Arg Lys Met Asp
    130                 135                 140

Thr Arg Thr Glu Val Asp Asp Ile Leu Asn Ser Ile Thr Glu Pro Arg
145                 150                 155                 160

Arg Leu Thr Leu Pro Gly Ile Asn Lys Met Arg Gln Cys Ile Val Arg
                165                 170                 175

Leu Leu Leu Leu Val Pro Ile Gln Val Arg Glu Glu Ile Leu Ser Phe
            180                 185                 190
```

Ala Ile Ala Ser Gly Ile Pro Ser Glu Thr Ile Glu Asp Ile Arg Ser
195 200 205

Ser Thr Asn Ile Ser Ala Val Asp Thr Asn Gly Arg Gly Ile Ala His
210 215 220

Asn Ser Lys Lys Arg Ser Leu Ala Pro Thr Gln Asp Ser Arg Asn Leu
225 230 235 240

Arg Arg Arg Ile Arg Gly His Thr Gln
245

<210> 78
<211> 453
<212> DNA
<213> Saccharomyces cerevisiae

<220>
<221> promoter
<222> (1)..(450)
<223>

<220>
<221> misc_feature
<222> (451)..(453)
<223> start codon

<400> 78

ctacttattc ccttcgagat tatatctagg aacccatcag gttggtggaa gattacccgt 60
tctaagactt ttcagcttcc tctattgatg ttacacctgg acaccccttt tctggcatcc 120
agtttttaat cttcagtggc atgtgagatt ctccgaaatt aattaaagca atcacacaat 180
tctctcggat accacctcgg ttgaaactga caggtggttt gttacgcatg ctaatgcaaa 240
ggagcctata tacctttggc tcggctgctg taacagggaa tataaagggc agcataattt 300
aggagtttag tgaacttgca acatttacta ttttcccttc ttacgtaaat atttttcttt 360
ttaattctaa atcaatcttt ttcaattttt tgtttgtatt cttttcttgc ttaaatctat 420
aactacaaaa aacacataca taaactaaaa atg 453

<210> 79
<211> 499
<212> DNA
<213> Zygosaccharomyces bailii

<220>
<221> promoter
<222> (1)..(496)
<223>

<220>

<221> misc_feature
<222> (497)..(499)
<223> start codon

<400> 79

```
ggatcgtatt gcttccattc ttcttttgtt attcggcgcg attcgaattc atgacatctt    60
ttaaccgtcc gcactacatt actggctcaa gaaaggattg ataaatacta ccaaggaaca   120
cgtgtatcca tttgatactg tgctggttac aagacacatg ctttacaagc acacttctat   180
ctctctcgac tgaggcgaaa cgtcgagtgg tttgatatca aatgcatgcg tgatatgcac   240
cattattttt cccttttact tccgtcacgc cggggctcca ctttttttggg ttccactttt   300
cttacgaccc tcgacatcca ctaaacgaac aggaagtcaa agaacccctc gagtcacacg   360
gtgcgtatgc gctgttaaca tatataaagg tcacctttcc ctgctcaaaa gagtcttagc   420
aggctgttaa cttcactctc tatcgatcca tagaatctaa ctaacaagag actacatcgg   480
tataacaaat aacaaaatg                                                499
```

<210> 80
<211> 27
<212> DNA
<213> artificial sequence

<220>
<223> PCR primer

<400> 80
aagagactcc aacgtcgcgc acctgta 27

<210> 81
<211> 32
<212> DNA
<213> artificial sequence

<220>
<223> PCR primer

<400> 81
agaggattag gaagacacaa attgcatggt ga 32

<210> 82
<211> 29
<212> DNA
<213> artificial sequence

<220>
<223> PCR primer

<400> 82
atcgtattgc ttccattctt cttttgtta 29

<210> 83
<211> 29

<212> DNA
<213> artificial sequence

<220>
<223> PCR primer

<400> 83
tttgttattt gttataccga tgtagtctc 29

<210> 84
<211> 27
<212> DNA
<213> artificial sequence

<220>
<223> PCR primer

<400> 84
tagctactct tctccaggtg tcattag 27

<210> 85
<211> 25
<212> DNA
<213> artificial sequence

<220>
<223> PCR primer

<400> 85
cctatgtccg agtttagcga gcttg 25

<210> 86
<211> 25
<212> DNA
<213> artificial sequence

<220>
<223> PCR primer

<400> 86
agaatgaact cagagttctc tcttg 25

<210> 87
<211> 22
<212> DNA
<213> artificial sequence

<220>
<223> PCR primer

<400> 87
attctattgg gtatgtcccc tg 22

<210> 88
<211> 30
<212> DNA
<213> artificial sequence

<220>
<223> PCR primer

<400> 88
gtttttaatt ttgaagctca cctttaattg 30

<210> 89
<211> 26
<212> DNA
<213> artificial sequence

<220>
<223> PCR primer

<400> 89
attatgttct ccagggaaga ggttag 26

<210> 90
<211> 27
<212> DNA
<213> artificial sequence

<220>
<223> PCR primer

<400> 90
agaatcaatc atttagtgtg gcaggag 27

<210> 91
<211> 25
<212> DNA
<213> artificial sequence

<220>
<223> PCR primer

<400> 91
taaaaactgc ccgccatatt tcgtc 25

<210> 92
<211> 708
<212> DNA
<213> Zygosaccharomyces rouxii

<400> 92

```
ctgcagaaag ccctaagatg ctcctcccgt tcacatgctc cgaacccttt ggaaaattct      60
gtgcgcggcg ctagcacgta atgacccttg atgacaaact ccaatggtat caccctactg     120
tcctctcccc ctccccttttt tttccttctt tctttccatc tatttctgat ctcctcccct    180
cagcagatgt cccgaaaggt acagctgcga tacgggcagc cactttttga cgtctcgcaa     240
caggatcacc ctgcacgacg gggcacaata ggattcccgt tggcacggtg ctggtgtata     300
gccgccgagg gtggggtata aagggctaca tccttacccc cacgcaggcg ataacccgca     360
tcatacaact gtcctcctct tccgctctcg ccactagccg ccgaaccatt gctaccgcaa     420
tgacaccgtg tggtgatctc aagggaggat gtgtgggtgt gggacggaac ttccactttt     480
tcctcagtag gtgcgatgcc ccctacaccg agcttccact aacgtgtttc agcggttgaa     540
ggcaatggga tcgcagaatt atcgcagctt gttggtatat aaagggagaa gatatatgga     600
taagagacat gttctacttc tgttctctct ttctttttat cctatatcac cagaacaaat     660
caagttcgca ttgattcata tcaaataaaa agtacatcac agataaca                  708
```

<210> 93
<211> 21
<212> DNA
<213> artificial sequence

<220>
<223> PCR primer

<400> 93
TGCAGAAAGC CCTAAGATGC T 21

<210> 94
<211> 29
<212> DNA
<213> artificial sequence

<220>
<223> PCR primer

<400> 94
TGTCTGTGAT GTACTTTTTA TTTGATATG 29

<210> 95
<211> 25
<212> DNA
<213> artificial sequence

<220>
<223> PCR primer

<400> 95
ACGCAAGAGA GAACTCTGAG TTCAT 25

## Description

High level production of proteins from engineered organisms (recombinant, mutagenised, ...) provides an alternative to the extraction of the proteins from natural sources. Natural sources of proteins are often limited, and furthermore the concentration of the desired product is generally low so extraction is regularly very cost-intensive or even impossible. Besides, extraction might bear the danger of toxic or infectious contamination depending on the natural origin of the protein.

With the advent of molecular cloning in the mid-70s, it became possible to produce foreign proteins in new hosts. Recombinant DNA (rDNA) technologies (genetic, protein and metabolic engineering) allow the production of a wide range of peptides and proteins from naturally-non producing cells. In fact the first biotech-products on the world market made by means of rDNA were pharmaceutical products (for example insulin, interferons, erythropoietin, vaccine against hepatitis B) and industrial enzymes (for example used for the treatments of food, feed, detergents, paper-pulp and health care). World-sales of the top-20 recombinant pharmaceutical products in 2000 was about 13 billions Euro, while the world-wide market for the industrial enzymes was about 2.0 and it is projected to reach about 8 billions Euro in 2008.

Microorganisms as well as cultured cells from higher organisms (such as mammalians, insects or plants) represent the mainly conceivable hosts for the production of heterologous as well as homologous proteins.

Several processes using mammalian cell culture for the production of proteins have been developed and many in such a manner produced proteins are on the market. Among them, several vaccines, monoclonal antibodies, interferon, blood factors, urokinase and tPA, hormones and growth factors.

The main advantage of a mammalian cell based expression system is the ability of mammalian cells to process the proteins in a proper way (correct folding, appropriate post-translational modification, correct glycosylation, specific proteolytic activities, etc.). A cloned protein expressed from recombinant DNA of mammalian origin (human) is usually correctly processed and folded and commonly secreted into the medium, allowing a fast recovery and purification.

On the other hand the costs of production are generally quite high due to a usually low level of expression, costs of the mammalian medium components, very slow growth rates and demanding culture conditions. Furthermore, production in mammalian cells bears the danger of toxic or infectious contamination of the product.

Microorganisms (prokaryotic as well as eukaryotic) are advantageous hosts for the production of proteins because of high growth rates and commonly ease of genetic manipulation. But, in particular, bacterial hosts lack the ability of a correct protein processing and in a lot of cases heterologously produced proteins build up inclusion bodies inside of the bacterial cells, whereupon the proteins are lost, because their enzymatic activity can in most instances not be reconstituted. Due to their incorrect structure any use of such proteins for the treatment of humans is also excluded.

Yeast hosts can combine the advantages of unicellular organisms (i.e., ease of genetic manipulation and growth) with the capability of a protein processing typical for eukaryotic organisms (i.e. protein folding, assembly and post-translational modifications), together with the absence of endotoxins as well as oncogenic or viral DNA. Starting from the early 80s, the majority of recombinant proteins produced in yeast have been expressed using *Saccharomyces cerevisiae* (Hitzeman, R. A. et al., 1981, Nature 293, 717-22). The choice was determined by the familiarity of molecular biologists to this yeast together with the accumulated knowledge about its genetics and physiology. Furthermore, *S. cerevisiae* is an organism generally regarded as safe (GRAS). However, this yeast is not an optimal host for the large-scale production of foreign proteins, especially due to its characteristics regarding fermentation needs. In particular, growth of *S. cerevisiae* shows a very pronounced Crabtree effect, therefore fed-batch fermentation is required to attain high-cell densities (see for example Porro, D., et al., 1991, Res. Microbiol. 142, 535-9). Furthermore, this yeast is comparatively sensitive regarding the culture conditions, for example regarding the pH value and the temperature. Therefore, its cultivation is complicated and requires a highly sophisticated equipment. In addition, the proteins produced by S. *cerevisiae* are often hyper-glycosylated and retention of the products within the periplasmic space is frequently observed (Reiser, J. et al., 1990, Adv. Biochem. Eng./Biophys. 43, 75-102 and Romanos, M. A. et al., 1992, Yeast 8, 423-88). Furthermore, due to the partial retention of the protein in S. *cerevisiae,* a fraction of the protein is commonly degraded. These respective degradation products are generally very difficult to remove from the desired product. Disadvantages such as these have promoted a search for alternative hosts, trying to exploit the great biodiversity existing among the yeasts, and starting the development of expression systems in the so-called "non conventional" yeasts. Prominent examples are *Hansenula polymorpha* (Buckholz, R. G. et al., 1991, Bio/Technology 9, 1067-72); *Pichia pastoris* (Fleer, R., 1992, Curr. Opin. Biotechnol. 3, 486-96); *Kluyveromyces lactis* (Gellissen, G. et al., 1997, Gene 190, 87-97); *Yarrowia lipolytica* (Muller, S. et al., 1998, Yeast 14, 1267-83) among others. Another yeast genus under investigation is the genus *Zygosaccharomyces.* Eleven species, which appear to be evolutionary quite close to *S. cerevisiae* and not so far from *K. lactis* have been classified so far (James, S. A. et al., 1994, Yeast 10, 871-81, Steels, H., et al., 1999, Int. J. Syst. Bacteriol. 49, 319-27 and Kurtzman, C. P., et al., 2001, FEMS Yeast research 1, 133-8). An exceptional resistance to several stresses renders some of the *Zygosaccharomyces* species potentially interesting for industrial purposes. For example *Z. rouxii* is known to be salt tolerant (osmophilic) and *Z. bailii* is known to tolerate high sugar concentrations and acidic environments as well as relatively high temperatures of growth (Makdesi, A. K. et al. 1996, Int. J. Food Microbiol. 33, 169-81 and Sousa, M. J. et al., 1996, Appl. Environm. Microbiol. 62, 3152-7). However, the data available related to the molecular biology of these yeasts are very poor. While expression and secretion of a heterologous protein could be achieved in *Z. rouxii* (Ogawa, Y. et al. 1990, Agric. Biol. Chem. 54, 2521-9), for *Z. bailii* just the first molecular tools to successfully transform this yeast and to express heterologous proteins intracellulary have been developed (WO 00/41477). Since purification of intracellular proteins is very elaborate, the use of this host for industrial production processes remains limited. Furthermore, while a lot of such non-conventional yeasts show specific advantages regarding their cultivation requirements, a lot of times these advantages are foiled by unexpected negative characteristics or unsolvable problems in their handling. In a lot of instances the tools for transformation of the organisms or expression of heterologous genes are not developed or the development fails due to unfavourable natural properties of the organism in question. The secretory capabilities often impose further problems for the production of proteins in industrial scale. If the organism does not allow the efficient secretion of the desired protein, the isolation of the product is significantly complicated. In addition, some very interesting products, such as Interleukin 1-β, turned out to be toxic for the cells as long as they are intracellulary located (Fleer, R. et al., 1991, Gene 107, 285-95). Production of such proteins is therefore only possible if the host comprises a highly potent secretory system that can be exploited. Another problem come from a potentially different codon usage or codon frequency that can hamper the expression of heterologous genes in such organisms decisively.

In consideration of the state of the art, the problem to be solved by the present invention was to provide a new, easy and economical method for the production of proteins. Apart of being cost effective that method should be easy to perform and allow the production of highly pure proteins in a high yield.

This problem as well as all further not explicitly mentioned problems, that are easily deduced from the introductory explicated contents, are solved by the objects outlined in the claims of the instant invention.

An advantageous process for the production of a protein is provided by a method as outlined in claim one. This method comprises culturing a *Zygosaccharomyces bailii* strain containing a vector comprising the DNA sequence coding for the protein functionally linked to a signalling sequence selected from the group consisting.of the signalling pre-sequence of the alpha-subunit of the Kl killer toxin of *Kluyveromyces lactis* and the signal sequence of the pre-pro α-factor of *Saccharomyces cerevisiae,* and further functionally linked to a promoter expressing and secreting the protein and isolating the protein. This process is particularly advantageous in that Z *bailii* can be cultured yieldingly in a chemically defined medium without the addition of complex ingredients that have to be separated tediously from the protein produced. Surprisingly, the secretory capacity of this yeast in chemically defined medium is significantly superior to the secretory capacity of S. *cerevisiae* under identical conditions. A further important advantage is the surprising fact that the protein produced by *Z. bailii* is not only readily secreted but also near to completion, what is not the case for *S. cerevisiae* under identical conditions. Through efficient secretion of the desired protein by *Z. bailii* also no degradation of the protein takes place. Subsequently, the purification of the product is significantly simplified.

Further major advantages of *Z. bailii* as host organism for protein production, and in particular for production of heterologous proteins are a naturally favourable codon usage as deduced from the examples presented herein and the comparatively low demands on the culture conditions. This is in particular due to a high acid and temperature tolerance as well as a weak Crabtree effect allowing the cultivation with a high sugar concentration from the beginning (i.e. batch instead of fed-batch cultivation) and the omission of extremely sophisticated regulations of the culture conditions such as temperature or pH. Accordingly, this method allows a cost effective production of proteins in an easy way even in industrial scale yielding proteins of high purity.

The term "expression" of a protein by a host cell is well known to the skilled artisan. Usually expression of a protein comprises transcription of a DNA sequence into a mRNA sequence followed by translation of the mRNA sequence into the protein. A more detailed description of the process can be found for example in Knippers, R. et al, 1990, Molekulare Genetik, Chapter 3, Georg Thieme Verlag, Stuttgart.

The term "secretion" of a protein as known in the art means translocation of the protein produced, from inside of the cell to outside of the cell, thereby accumulating the protein in the culture medium. A more detailed description of the process can be found for example in Stryer, L., 1991, Biochemie, Chapter 31, Spektrum Akad. Verlag, Heidelberg, Berlin, New York.

The protein produced might be any protein known in the art for which an industrial production is desirable. For example the protein might be useful in the pharmaceutical field, such as medication or vaccine or in pre-clinical or clinical trials among others (examples are growth hormones, tissue plasminogen activator, hepatitis B vaccine, interferones, erythropoietin). The protein produced might also be useful in industry for example in the area of food production (e.g. β-galactosidase, chymosin, amylases, glucoamylase, amylo-glucosidase, invertase) or textile and paper production (proteases, amylases, cellulases, lipases, catalases, etc.). Enzymes are useful among others as detergents (proteases, lipases and surfactants) and their characteristics of stereo-specificity are furthermore exploitable in a wide number of bioconversions, yielding a desired chiral compound. Another promising application of recombinant enzymes that can be produced by the method of the instant invention is the development of biosensors.

The proteins secreted can vary greatly in size (molecular weight). The herein described method functions well for very small proteins (e. g. IL-1β, 17 kDa, see Fig. 5), but also for quite large proteins (e.g. GAA, 67.5 kDa, see Fig. 8a). The secreted proteins may or may not comprise consensus sites for glycosylation. Such consensus sites might occur naturally or might be introduced by genetic engineering. Depending on the intended use of the protein produced it might also be advantageous to remove naturally occurring consensus sites for glycosylation by genetic engineering, thereby preventing for example hyper-glycosylation of the protein. Remarkably, the herein described method leads to proteins that conserve their desired catalytic characteristics after the secretion (e.g. GAA, see Fig. 8a).

In the present invention the *Z. bailii* strain is transformed with a vector comprising a DNA sequence coding for the protein, functionally linked to a signal sequence leading to the secretion of the protein and further functionally linked to a promoter leading to the expression of the protein.

The term "vector" refers to any agent as such a plasmid, cosmid, virus, phage, or linear or circular single-stranded or double-stranded DNA or RNA molecule, derived from any source that carries nucleic acid sequences into a host cell. Preferably a vector is capable of genomic integration or autonomous replication. Such a vector is capable of introducing a 5' regulatory sequence or promoter region and a DNA sequence for a selected gene product into a cell in such a manner that the DNA sequence is transcribed into a functional mRNA, which may or may not be translated and therefore expressed. Preferably the vector is an extra-chromosomal plasmid. Such a plasmid comprises preferably an autonomously replicating sequence (ARS) and advantageously a centromeric sequence (CEN) in addition. More preferable the plasmid is a 2μ-like episomal multicopy plasmid. Even more preferably the plasmid is derived from an endogenous episomal plasmid from a *Z. bailii* strain such as pSB2 (Utatsu, I. et al., 1987, J. Bacteriol. 169, 5537-45) and more preferably from $pZB_1$ or $pZB_5$ (see Fig. 9).

The plasmid $pZB_5$ was extracted from NCYC 1427 and partially sequenced. Accordingly, the plasmid comprises preferably at least 35, more preferably at least 55 and even more preferably at least 75 and even more preferably at least 100 bases from at least one of the sequences selected from the list of SEQ ID No.: 63, SEQ ID No.: 64, SEQ ID No.: 65, SEQ ID No.: 66, SEQ ID No.: 67, SEQ ID No.: 68, SEQ ID No.: 69, SEQ ID No.: 70 or SEQ ID No.: 71.

Yeast multicopy plasmids (also referred to as 2μ or 2μm-like plasmids) isolated from different yeast genus or species usually show a well conserved structural homology while having a low sequence homology. Some regulatory elements were identified as necessary and sufficient to build a functional multicopy plasmid. These are:

the recombinase promoting amplification of these plasmids, encoded by the *FLP* gene. (Blanc H., et al., 1979, Mol. Gen. Genet. 176, 335-42 and Broach J.R. et al., 1980, Cell 21, 501-8);

two inverted repeats (IR-sequences);

a single origin of replication (*ARS*) at the junction between an internal repeat and a unique region of the plasmid (Broach J. R. et al., 1980, Cell 21, 501-8; Brewer B. J. et al., 1987, Cell 51, 463-71; McNeil J. B., et al., 1980, Curr. Genet. 2, 17-25) and

the regulatory proteins *REP1*/*REP2* (in *Z. bailii* referred to as *TFB*/*TFC),* controlling the amplification process, by limiting the recombinase activity in the cell through-mediated repression of *FLP* gene expression (Broach J. R. et al., 1980, Cell 21, 501-8; Jayaram M. et al., 1983, Cell 34, 95-104).

Within the scope of the instant invention these key elements of the 2μ plasmid are preferably derived from *Z. bailii,* even more preferably from *Z. bailii* NCYC1427 or ATCC36947. Particularly preferred these sequences correspond to SEQ ID No.: 71 (IR-ARS), SEQ ID No.: 72 *(FLP),* SEQ ID No.: 74 *(TFB)* and SEQ ID No.: 76 (*TFC*)*,* respectively. The expressed recombinase and the expressed regulatory proteins exhibit preferably the amino acid sequence shown in SEQ ID No.: 73 *(FLP),* SEQ ID No.: 75 *(TFB)* and SEQ ID No.: 77 (*TFC*)*,* respectively. Preferably the plasmid additionally comprises the homologue upstream regions of the *FLP* and the *TFB*/*TFC* genes, in order to obtain an optimal control of the transcription level.

Generally speaking the plasmid preferably comprises sequences for (autonomous) replication, stabilization and/or plasmid copy number control, obtainable from a *Z. bailii* strain.

Preferably the plasmid is $pEZ_1$ (see Fig. 9c)

Particularly preferred is the plasmid $pEZ_2$ (see Fig. 9d). One preferred way to construct $pEZ_2$ is to amplify the IR/ARS region and the TFC/FLP genes including their homologous promoters by PCR with the oligos

5'-AGAATCAATCATTTAGTGTGGCAGGAG-3' (SEQ ID NO.: 90) and

5'-TAAAAACTGCCCGCCATATTTCGTC-3' (SEQ ID NO.: 91, *IRAARS*),

5'-AGAATGAACTCAGAGTTCTCTCTTG-3' (SEQ ID NO.: 86) and

5'-CCTATGTCCGAGTTTAGCGAGCTTG-3'(SEQ ID NO.: 85, *FLP*/*TFC*)

and to substitute the ARS/CEN cassette from $pZ_3$ with these amplified products. Another way is to substitute the 2μ-ori sequence from the plasmid p195 with the aforementioned PCR-products.

Advantageously, the vector comprises a selectable marker. The term selectable marker refers to a nucleic acid sequence whose expression confers a phenotype facilitating identification of cells containing the nucleic acid sequence. Selectable markers include those which confer resistance to toxic chemicals (= dominant marker, e.g. G418, hygromycin, formaldehyde, phleomycin or fluoroacetate like reviewed in Van den Berg, M. et al, 1997, Yeast 13, 551-9) or complement an auxotrophy (=auxotrophic marker, e.g. uracil, histidine, leucine, tryptophane). Auxotrophic selection markers can be used for naturally auxotrophic *Z. bailii* strains or strains that have been rendered auxotrophic by genetical manipulation, in particular by (partial) deletion or mutagenisation of an essential gene, e.g. *HIS3* (Branduardi, P., 2002, Yeast 19, 1165-70). As complementing marker sequence the homologous gene from *Z. bailii* or a heterologous gene might be employed. Auxotrophic markers are preferred since no component has to be added to the medium to keep the selective pressure during the cultivation.

The term "promoter" or "promoter region" refers to a DNA sequence, usually found upstream (5') to a coding sequence, that controls expression of the coding sequence by controlling production of messenger RNA (mRNA) by providing the recognition site for RNA polymerase and/or other factors necessary for start of transcription at the correct site. The promoter can be derived from any organism. Preferably the promoter is derived from a yeast, even more preferably from *Saccharomyces, Kluyveromyces* or *Zygosaccharomyces* and most preferably from *Z. rouxii or Z. bailii.* The promoter can be constitutive, inducible or repressible. Inducible promoters can be induced by the addition to the medium of an appropriate inducer molecule or by an appropriate change of the chemical or physical growth environment (such as the temperature or pH value), which will be determined by the identity of the promoter. Repressible promoters can be repressed by the addition to the medium of an appropriate repressor molecule or by an appropriate change of the chemical or physical growth environment (such as the temperature or pH value), which will be determined by the identity of the promoter. Constitutive promoters are preferred, as the use of an appropriate repressor or inducer molecule or an appropriate change of the chemical or physical growth environment is not required. Preferably the promoter is selected from the list of: triose-phosphate isomerase (TPI), glyceraldehyde phosphate dehydrogenase (GAPDH), alcohol dehydrogenase 1 (ADH1), phosphoglycerate kinase (PGK), glyceraldehyde-3-phosphate dehydrogenase (GAP), GAL1, GAL10, acid phosphatase (PHO5), cytochrome C-1 (CYC1), copper-binding metallothionein (CUP1) or a-factor mating pheromone precursor (Mfa1) promoter or the hybrid promoters GAL/CYC1, such as GAL1-10/CYC1, GAP/GAL, PGK/GAL, GAP/ADH2, GAP/PHO5 or CYC1/GRE either from *S. cerevisiae, Z. rouxii* or *Z. bailii,* but preferred from *Z. bailii.* Especially preferred promoters are the TPI promoters either from S. *cerevisiae* corresponding to SEQ ID No.: 78 or *Z. bailii* corresponding to SEQ ID No.: 79, but particularly preferred is the TPI promoter from *Z. bailii* (SEQ ID No.: 79). Further particularly preferred promoters are the GAPDH promoters from *Z. rouxii* (SEQ ID No.: 92) or *Z. bailii*.

Furthermore the vector comprises preferably a transcriptional terminator sequence following the coding sequence for the desired protein for efficient mRNA 3 end formation. Such a terminator sequence is preferably derived from a yeast, more preferably from *Saccharomyces* or *Zygosaccharomyces,* even more preferably from S. *cerevisiae* or *Z. bailii* and most preferably from *Z. bailii.* A preferred example for a terminator sequence comprises the following tripartite consensus sequence: TAG..(T-rich)..TA(T)GT..(AT-rich)..TTT. Another preferred example comprises the sequence motif TTTTTATA.

Further the vector comprises a signalling sequence (=leader sequence; upon expression translated into signal peptide or leader peptide). Such sequences lead to the direction of expressed proteins from the cytosol into the culture medium. In other words signal sequences cause the secretion of the proteins and their accumulation in the medium. Signal sequences generally code for a continuous stretch of amino acids, typically 15 to 60 residues long (up to 150), which characteristically include one or more positively charged amino acid(s) followed by a stretch of about 5 to 10 hydrophobic amino acids, which may or may not be interrupted by non-hydrophobic residues. Preferably the signal peptide comprises 15-45 amino acids, even more preferably 15 to 30 amino acids. Even though their amino acid sequences can vary greatly, the signal peptides of all proteins having the same destination in one organism are functionally interchangeable: physical properties, such as hydrophobicity or the pattern of charged amino acids, often appear to be more important in the signal-recognition process than the exact amino acid sequence.

Preferably the DNA sequence coding for the signal peptide is selected from the list of: SEQ ID NO.: 1, SEQ ID NO.: 3, SEQ ID NO.: 5, SEQ ID NO.: 7, SEQ ID NO.: 9, SEQ ID NO.: 11, SEQ ID NO.: 13, SEQ ID NO.: 15, SEQ ID NO.: 17, SEQ ID NO.: 19, SEQ ID NO.: 21, SEQ ID NO.: 23, SEQ ID NO.: 25, SEQ ID NO.: 27, SEQ ID NO.: 29, SEQ ID NO.: 31, SEQ ID NO.: 33, SEQ ID NO.: 35, SEQ ID NO.: 37, SEQ ID NO.: 39, SEQ ID NO.: 41, SEQ ID NO.: 43, SEQ ID NO.: 45, SEQ ID NO.: 47, SEQ ID NO.: 49, SEQ ID NO.: 51, SEQ ID NO.: 53, SEQ ID NO.: 55, SEQ ID NO.: 57, SEQ ID NO.: 59, SEQ ID NO.: 61. Even more preferably the amino acid sequence of the signal peptide is selected from the list of: SEQ ID NO.: 2, SEQ ID NO.: 4, SEQ ID NO.: 6, SEQ ID NO.: 8, SEQ ID NO.: 10, SEQ ID NO.: 12, SEQ ID NO.: 14, SEQ ID NO.: 16, SEQ ID NO.: 18, SEQ ID NO.: 20, SEQ ID NO.: 22, SEQ ID NO.: 24, SEQ ID NO.: 26, SEQ ID NO.: 28, SEQ ID NO.: 30, SEQ ID NO.: 32, SEQ ID NO.: 34, SEQ ID NO.: 36, SEQ ID NO.: 38, SEQ ID NO.: 40, SEQ ID NO.: 42, SEQ ID NO.: 44, SEQ ID NO.: 46, SEQ ID NO.: 48, SEQ ID NO.: 50, SEQ ID NO.: 52, SEQ ID NO.: 54, SEQ ID NO.: 56, SEQ ID NO.: 58, SEQ ID NO.: 60, SEQ ID NO.: 62.

Particularly preferred the DNA sequence coding for the signal peptide is selected from the list of SEQ ID NO.: 1, SEQ ID NO.: 3, SEQ ID NO.: 21 or SEQ ID NO.: 35 correspondingly the amino acid sequence of the signal peptide is preferably selected from the list of SEQ ID NO.: 2, SEQ ID NO.: 4, SEQ ID NO.: 22 or SEQ ID NO.: 36.

The signal peptide is preferably removed from the finished protein. This can occur through activity of a specialised signal peptidase. The signal peptidase can be of homologous or heterologous origin. Therefore, the signal peptide comprises preferably a processing site or a cleavage site that allows for recognition by a specific endopeptidase.

In a preferred embodiment of the present invention the *Z. bailii* strain is transformed with a vector comprising the DNA sequence coding for the protein, functionally linked to the signalling pre-sequence (16 aa) of the alpha-subunit of the K1 killer toxin of *K. lactis* (Stark M.J. et al., 1986, EMBO J. 5,1995-2002, SEQ ID NO.: 35 (DNA) and SEQ ID NO.: 36 (peptide)) and further functionally linked to the TPI promoter from S. *cerevisiae.* More preferably the vector is $pZ_3kl$ (Figure 1b). Even more preferably the *Z. bailii* strain is transformed with a vector comprising the DNA sequence coding for the protein, functionally linked to the signal sequence of the K1 killer toxin of *K. lactis* and further functionally linked to the GAPDH promoter from *Z. rouxii.* Even more preferably the *Z. bailii* strain is transformed with a vector comprising the DNA sequence coding for the protein, functionally linked to the signal sequence of the K1 killer toxin of *K. lactis* and further functionally linked to the TPI promoter from *Z. bailii.* Particularly preferred said vector is derived from $pZ_3bT$ (Figure 4a).

In another preferred embodiment of the present invention the *Z. bailii* strain is transformed with a vector comprising the DNA sequence coding for the protein, functionally linked to the signal sequence of the pre-pro α-factor of S. *cerevisiae* and further functionally linked to the TPI promoter from *S. cerevisiae.* Preferably the vector is $pZ_3pp\alpha$ (Figure 1c). Even more preferably the *Z. bailii* strain is transformed with a vector comprising the DNA sequence coding for the protein, functionally linked to the signal sequence of the pre-pro a-factor of S. *cerevisiae* and further functionally linked to the GAPDH promoter from *Z. rouxii.* Even more preferably the *Z. bailii* strain is transformed with a vector comprising the DNA sequence coding for the protein, functionally linked to the signal sequence of the pre-pro a-factor of *S. cerevisiae* and further functionally linked to the TPI promoter from *Z. bailii.* Particularly preferred said vector is derived from $pZ_3bT$ (Figure 4a).

In yet another preferred embodiment of the present invention the *Z. bailii* strain is transformed with a vector comprising the DNA sequence coding for the protein, functionally linked to the zygocin killer toxin pre-sequence of *Z. bailii* (SEQ ID No.: 59) and further functionally linked to a promoter functional in *Z. bailii.* Preferably said promoter is the TPI promoter from *S. cerevisiae.* Even more preferably said promoter ist the TPI promoter from *Z. bailii.* Most preferred is the GAPDH promoter from *Z. rouxii.*

The DNA sequence coding for the protein can be derived from animal, bacterial, fungal, plant or viral sources, more preferably from metazoan, mammalian or fungal sources. The expressed protein might therefore be homologous or heterologous to *Z. bailii.*

Any yeast belonging to the species *Z. bailii* can be used for the production of proteins in the scope of the present invention. In a preferred embodiment of the invention the *Z. bailii* strain is transformed. "Transformation" refers to a process of introducing an exogenous nucleic acid sequence (of homologous and/or heterologous origin, recombinant or not) into a cell in which that exogenous nucleic acid is incorporated into a chromosome or is capable of autonomous replication. A cell that has undergone transformation, or a descendant of such a cell, is "transformed" or "recombinant". If the exogenous nucleic acid comprises a coding region encoding a protein and the protein is produced in the transformed yeast such a transformed yeast is functionally transformed. Preferred methods to transform *Z. bailii* are electroporation, as described in [WO 00/41477], or the chemical LiAc/PEG/ssDNA method as described by Agatep, R. et al., 1998, Technical Tips Online (http://tto.trends.com).

Preferably the *Z. bailii* strain that is being transformed is selected from the list of: ATCC 36947, ATCC 60483, ATCC 8766, FRR 1292, ISA 1307, NCYC 128, NCYC 563, NCYC 1416, NCYC 1427, NCYC 1766, NRRL Y-2227, NRRL Y-2228, NRRL Y-7239, NRRL Y-7254, NRRL Y-7255, NRRL Y-7256, NRRL Y-7257, NRRL Y-7258, NRRL Y-7259, NRRL Y-7260, NRRL Y-7261, NRRL Y-27164; particularly preferred are ATCC 36947, ATCC 60483, ATCC 8766 and NCYC 1427.

(ATCC: American Type Culture Collection, Manassas VA, USA; FRR: FRR Culture Collection, North Ryde NSW, Australia; ISA: Culture Collection of the Instituto Superior de Agronomia, Lisbon; NCYC: National Collection of Yeast Cultures, Norwich, UK; NRRL: Agricultural Research Service Culture Collection, Peoria IL, USA).

Within the scope of the present invention the *Z. bailii* strain can be subjected to a selection process for improved secretion. Screening for and isolation of such a "super-secreting" phenotype can occur before or after transformation of the respective *Z. bailii* strain.

In a preferred embodiment of the present invention the *Z. bailii* gene/s homologous to *GAS1* from *S. cerevisiae* are identified and disrupted. *GAS1* is one example for the few cases wherein the key molecules involved in the intriguingly complex secretory pathway have been identified. It was possible to influence the whole secretory mechanism modifying the Gas1 expression level in *S. cerevisiae* (Vai, M., et al., 2000, Appl. Environ. Microbiol. 66, 5477-9) due to a resultant modification of the organisation of the cell wall structure, namely it was demonstrated that *gas1* mutants show a "super-secreting" phenotype (Popolo L., et al., 1997, J. Bacteriol. 180, 163-6; Ram A. F. J., et al., 1998, J. Bacteriol. 180, 1418-24).

In another preferred embodiment of the present invention the *Z. bailii* strain has undergone one or more mutagenisation/selection cycle(s) to obtain super secreting mutants, comprising chemical or physical mutagenesis. Preferably the mutagenisation is caused by orthovanadate. Orthovanadate is a molecule known to affect the glycosylation process and the cell wall construction in *S. cerevisiae* (Kanik-Ennulat, C. et al., 1990, Mol. Cell. Biol. 10, 898-909). Methods involving orthovanadate mutagenisation to obtain cells with changed cell wall construction/secretory properties that are useful in the scope of the present invention are disclosed in more detail for example for *S. cerevisiae* (Willsky. G.R., et al., 1985, J. Bacteriol. 164, 611-7) and *K. lactis* (Uccelletti, D., et al., 1999, Res. Microbiol. 150, 5-12; Uccelletti. D., et al., 2000, Yeast 16, 1161-71).

Culturing techniques and media suitable for yeast are well known in the art. Typically, but it is not limited to, culturing is performed by aqueous fermentation in an appropriate vessel. Examples for a typical vessel for yeast fermentation comprise a shake flask or a bioreactor.

The culture is typically performed at a temperature between 20°C and 40°C, preferably between 25°C and 35°C and even more preferred between 28°C and 32°C.

The medium in which the *Z. bailii* strain is cultured can be any medium known in the art to be suitable for this purpose. The medium might contain complex ingredients or might be chemically defined. Chemically defined media are preferred. The medium comprises any component required for the growth of the yeast. In particular the medium comprises a carbon source, such as fructose, glucose or other carbohydrates (such as sucrose, lactose, D-galactose, or hydrolysates of vegetable matter, among others). Typically, the medium also comprises further a nitrogen source, either organic or inorganic, and optionally the medium may also comprise macro nutrients and/or micro nutrients such as amino acids; purines; pyrimidines; corn steep liquor; yeast extract; protein hydrolysates, such as peptone; vitamins (water-soluble and/or water-insoluble), such as B complex vitamins; or inorganic salts such as chlorides, hydrochlorides, phosphates, or sulphates of Ca, Mg, Na, K, Fe, Ni, Co, Cu, Mn, Mo, or Zn, among others. Antifoam might be added, if necessary. Further components known to one of ordinary skill in the art to be useful in yeast culturing or fermentation can also be included. The medium may or may be not buffered. A preferred medium comprises yeast extract, peptone and glucose (=YPD). A more preferred medium comprises yeast extract, peptone and fructose (=YPF). An even more preferred medium comprises glucose and Yeast Nitrogen Base (YNB, Difco Laboratories, Detroit, MI #919-15). Another even more preferred medium comprises fructose and YNB.

Particularly preferred is a medium comprising high fructose corn syrup as carbon source (for example Isosweet® 100 42% High Fructose (80% solids) or Isosweet® 5500 55% Fructose from Tate & Lyle PLC or IsoClear® 42% High Fructose Corn Syrup or IsoClear® 55% High Fructose Corn Syrup from Cargill, Inc.).

The compositions of preferred media for batch/fed batch cultivation of *Z. bailii* according to the instant invention are as follows: the batch phase medium comprises 4% w/V Glucose, 0.5% w/V $(NH_4)_2SO_4$, 0.05% w/V $MgSO_4$, 0.3% w/V $KH_2PO_4$, vitamins according to Verduyn, C., et al., 1992, Yeast 8, 501-17, wherein the final concentration of vitamins will be 3 times in respect to the indicated concentrations and trace elements according to Verduyn, C., et al., 1992, Yeast 8, 501-17, wherein the final concentration will also be 3 times in respect to the indicated concentrations. The pH control (value: pH 5) is performed by the addition of 2M KOH. The fed-batch medium comprises 50% w/V Glucose, 15.708 g/l $KH_2PO_4$, 5 g/l KCl, 5.831 g/l $MgSO_4$, 1,2 g/l $CaCl_2$, 1 g/l Yeast Extract, 0.4447 g/l NaCl, 1 g/l Glutamate, 0,05 g/l $ZnSO_4$, 0,04 g/l $CuSO_4$, 0,05 g/l $MnCl_2$, 0,001 g/l $CoCl_2$, 0.5 g/l myo-inositol, 0.1 g/l thiamine hydrochloride, 0.02 g/l pyridoxol hydrochloride, 0.04 g/l Ca-D(+)panthotenate, 0.004 g/l d-biotin, 0.09 g/l nicotinic acid. The pH control (value: pH 5) is performed by the addition of 2M $NH_4OH$.

In case of selection for the dominant G418 marker 200mg/l G418 is added to the respective medium.

The use of a defined medium, of which the components are adjusted to the needs of the organism is preferred. The purification of the protein is thereby significantly simplified.

Preferably, the pH of the culture medium ranges between 2 and 9, more preferably between 3 and 8 and even more preferably between 4 and 7. The pH can be regulated or partially regulated or not be regulated during the course of fermentation; accordingly the pH can be kept constant at a preferred value or can change during fermentation. A significant advantage of *Z. bailii* is its surprising capacity to grow as well as express and secrete proteins at low pH. Therefore, the demand of this organisms for a strictly controlled pH is not very pronounced.

The cultivation can take place in batch, fed-batch or continuous mode as is known to the ordinary skilled artisan.

During the course of the fermentation, the desired protein is expressed, properly processed (i.e. folded, modified, cut, etc.) and secreted (=accumulated in the medium). While the protein produced may be partially retained within the yeast cells it is preferred that a substantial amount of the protein is secreted. Even more preferred is that the protein is entirely secreted.

After culturing has progressed for a sufficient length of time to produce a desired concentration of the protein in the yeast and/or the culture medium, the protein is isolated. "Isolated," as used herein to refer to the protein, means being brought to a state of greater purity by separation of the protein from at least one other component of the yeast or the medium. Preferably, the isolated protein is at least about 80% pure as based on the weight, more preferably at least about 90% pure as based on the weight and even more preferably at least about 95% pure as based on the weight. Evidence of purity can be obtained by SDS-PAGE, 2D electrophoresis, IF, HPLC, mass spectrometry, capillary electrophoresis or other methods well known in the art.

"Purity" refers to the absence of contaminants in the final purified protein. Typical contaminants to be separated from the desired product are proteins, pyrogens, nucleic acids and more.

The protein is isolated from the culture medium, preferably without lysing of the cells. Such an isolation comprises purifying the protein from the medium. Purification can be achieved by techniques well-known in the art, such as filtration (e.g. microfiltration, ultrafiltration, nanofiltration), crystallisation or precipitation, centrifugation, extraction, chromatography (e.g. ion exchange, affinity, hydrophobic exchange), among others.

Upon removal of the cells, the culture broth might also directly serve as the product (e.g. enzyme solution), without further purification. The medium components can be adjusted appropriately prior to the cultivation.

If the protein is not completely secreted, the protein can also be isolated from both the yeast cells and the medium. Methods for lysing of the yeast cells are known in the art and comprise chemical or enzymatic treatment, treatment with glass beads, sonication, freeze/thaw cycling, or other known techniques. The protein can be purified from the various fractions of the yeast lysate by appropriate techniques, such as filtration (e.g. microfiltration, ultrafiltration, nanofiltration), crystallisation or precipitation, centrifugation, extraction, chromatography (e.g. ion exchange, affinity, hydrophobic exchange), among others.

Another embodiment of the present invention relates to a *Z. bailii* strain, expressing and secreting a heterologous protein.

The *Z. bailii* strain might be transformed with a vector comprising a DNA sequence coding for the heterologous protein, functionally linked to a signal sequence leading to the secretion of the protein and further functionally linked to a promoter.

Description of the Figures:

**Figure 1**:Expression and Secretion Vectors

Schematic maps of the plasmids constructed for expression of proteins in *Z. bailii:* ***a:*** $pZ_3$, (intracellular expression), ***b*** : $pZ_3$k1 (expression and secretion) and ***c*** : $pZ_3pp\alpha$ (expression and secretion).

**a)** $pZ_3$ : the backbone of the plasmid is the pYX022 *S. cerevisiae* expression plasmid (R&D Systems, Inc., Wiesbaden, D; the expression cassette is based on the constitutive *S. cerevisiae* TPI promoter and the corresponding polyA signal, as indicated in the Figure). The ARS/CEN fragment, from Ycplac33 (Gietz, R. D., et al., 1988, Gene 74, 527-34) ensures replication and stability of the plasmid, while the $Kan^R$ cassette, derived from pFA6-KanMX4 (Wach, et al., 1994, Yeast 10, 1793-808) allows a G418-based selection of the transformants.

**b)** $pZ_3$kl: a $pZ_3$ expression vector comprising the signal sequence of the *K. lactis* K1 killer toxin **(kl)** for leading the secretion of the protein of interest.

**c)** $pZ_3pp\alpha$: a $pZ_3$ expression vector comprising the pre-pro leader sequence of the *S. cerevisiae* pheromone $\alpha$-factor (pre-pro-$\alpha$F) for leading the secretion of the protein of interest.

(Amp= ampicillin resistance cassette; MCS= multi cloning site; colE1 ori; *E. coli* replication origin)

**Figure 2:** Expression and Secretion Vectors

Schematic maps of the plasmids constructed for expression and secretion of the human IL-1$\beta$ (Auron, E., et al., 1984, PNAS 81, 7907-11) and the GFP (Heim, R. et al., 1996, Curr. Biol. 6, 178-82) in *Z. bailii.*

**a)** $pZ_3$klIL- 1$\beta$: a $pZ_3$kl vector where the sequence encoding for the human IL-1$\beta$ was sub-cloned into the MCS.

**b)** $pZ_3pp\alpha$IL-1$\beta$: a $pZ_3pp\alpha$ vector where the sequence encoding for the human IL-1$\beta$ was sub-cloned into the MCS.

**c)** $pZ_3$ ppαGFP: a $pZ_3$ppα vector where the sequence encoding for the GFP was sub-cloned into the MCS.

**Figure 3:** Expression and Secretion Vectors
Schematic maps of the plasmids constructed for the expression of the *Arxula adeninivorans* glucoamylase (GAA, Genebank accession no: Z46901, Bui Minh, D., et al., 1996, Appl. Microbiol. Biotechnol. 44, 610-9) and of the bacterial β-galactosidase (from the plasmid pSV-β-galactosidase of Promega, Inc.; Genebank accession no.: X65335) in *Z. bailii.*

a) $pZ_3$GAA: a $pZ_3$ vector where the sequence encoding for the glucoamylase (GAA) was sub-cloned into the MCS.

b) $pZ_3$LacZ: a $pZ_3$ vector where the sequence encoding for the β-galactosidase was sub-cloned into the MCS.

**Figure 4**: Expression Vectors
Schematic maps of the plasmids constructed for the expression of proteins in *Z. bailii* based on the *Z. bailii TPI* promoter.

**a)** $pZ_3$*b*T: a $pZ_3$ vector where the S. *cerevisiae TPI* promoter was substituted by the *Z. bailii TPI* promoter.

**b)** $pZ_3$*b*TLacZ: a $pZ_3$*b*T expression vector where the sequence encoding for the β-galactosidase was sub-cloned into the MCS.

**Figure 5:** IL-1β secretion

**a)** Growth kinetics in minimal (YNB) and rich (YPD) medium, with glucose 5% (w/V) as a carbon source: the cellular growth was measured as optical density (OD 660nm, circles) and the residual glucose (g/l, squares) was evaluated. Comparison between *S. cerevisiae* (open symbols) and *Z. bailii* (full symbols).

**b)** Western Blot analyses performed on cellular extracts of S. *cerevisiae* and *Z. bailii* cells transformed with the plasmid $pZ_3$klIL-1β (expressing IL-1β preceded by the leader sequence from the *K. lactis* killer toxin) and with the corresponding empty plasmid ($pZ_3$), as a negative control. In the first lane a positive control (IL-1β, human recombinant *(E. coli),* Roche cat n° 1 457 756) was loaded. Samples were collected at the indicated times and from the indicated media, corresponding to the kinetics showed in (a). The loaded volumes were rectified for a corresponding OD value of 0.08. The blotted membranes were probed with an α-IL-1β polyclonal antibody.

**c)** as above, were the loaded samples represent the corresponding supernatant.

**d)** as above, were the samples were loaded with an equal volume of medium (30μl).

**Figure 6:** Leading of the pre-pro-α-factor signal sequence to the secretion of IL-1β and of GFP in *Z. bailii*

**a)** Western Blot analyses performed on cellular extracts (i) and on supernatants (*ii*) of *Z. bailii* and *S. cerevisiae* cells transformed with the plasmid $pZ_3$ppαIL-1β (and with the corresponding empty plasmid $pZ_3$) and growing on YPD medium (glucose 2% wN). Samples were taken at the indicated times. First lane: positive control (IL-1β, human recombinant *(E. coli),* Roche cat n° 1 457 756). The blotted membranes were probed with an α-IL-1β polyclonal antibody.
Western Blot analyses performed on cellular extracts (*iii*) and on supernatants (*iv*) of *Z. bailii* and *S. cerevisiae* cells transformed with the plasmid $pZ_3$ppαIL-1β (and with the corresponding empty plasmid $pZ_3$) and growing on YNB medium (glucose 5% w/V). Samples were taken at indicated times. First lane: positive control (IL-1β human recombinant (*E. coli)* Roche cat n° 1 457 756). The blotted membranes were probed with an α-IL-1β polyclonal antibody.
b) Western Blot analyses performed on cellular extracts *(cells)* and on supernatants *(sup)* of *Z. bailii* cells growing on YNB medium (glucose 2% w/V) transformed with the control plasmid $pZ_3$ (1st and 2nd lanes) and with the plasmid $pZ_3$ppαGFP (3rd and 4th lanes). The blotted membrane was probed with an α-GFP polyclonal antibody. An arrow indicates the expected positive signal.

**Figure 7:** Batch cultivations of *Z. bailii* cells comprising the $pZ_3$klIL-1 β expression plasmid on chemically defined medium in high sugar concentration

**a)** Culture OD (full circles), dry weight (open circles), glucose consumption (full squares) and ethanol production (open triangle).

**b)** Western Blot analyses performed on the growth medium (lane 2 to 5) and on the cell extracts (lanes 6 to 9) of *Z. bailii* cells. Samples were collected at the indicated times of the kinetics, and an equal volume (30μl for the supernatants and 15μl for the cell extracts, respectively) was loaded. The blotted membranes were probed with an α-IL-1β polyclonal antibody.

First lane: positive control (IL-1β human recombinant (*E. coli)* Roche cat n° 1 457 756).

**Figure 8:** Enzymatic activity of heterologous enzymes expressed in *Z. bailii* cells

**a)** Determination of the *A. adeninivorans* glucoamylase activity (mU/OD) present in the growth medium (YNB, glucose 2% w/V) of *Z. bailii* cells transformed with the plasmid $pZ_3$GAA (and the respective empty plasmid $pZ_3$, as a control). Three independent clones were analysed (Cl. 1, Cl. 3 and Cl. 5).

**b)** Determination of the β-galactosidase activity (Miller U/OD) in cell extracts of *Z. bailii* cells transformed with the plasmid $pZ_3$LacZ (two independent clones) and with the plasmid $pZ_3$*b*TLacZ (three independent clones), and the respective empty plasmid $pZ_3$ as a control. Cells were grown in YPD medium (glucose 2% w/V), and samples were collected at indicated times.

On the left panel the *Z. bailii* strain ATCC 36947, on the right panel the strain *Z. bailii* ATCC 60483 were tested, respectively.

**Figure 9:** Construction of a *Z. bailii* multicopy plasmid
Schematic maps of the endogenous plasmids isolated from *Z. bailii* ATCC 36947, named $pZB_1$ **(a)** and from *Z. bailii* NCYC 1427, named $pZB_5$ **(b).**

**c):** *Z. bailii* multicopy expression vector comprising the genes and the sequences necessary and sufficient for a stable and autonomous high copy number replication. The expression cassette is based on the *Z. bailii* constitutive *TPI* promoter and the polyA, as indicated in the Figure. The marker for selection is the $Kan^R$ cassette.

**d)** Z. *bailii* multicopy expression vector. The expression cassette is based on the *Z. bailii* constitutive TPI promoter and the polyA, as indicated in the Figure. Furthermore, the vector comprises the IR/ARS region and the TFC/FLP genes including their homologous promoters as indicated.

**Figure 10:** Influence of the promoter or the plasmid constituents, respectively, on β-galactosidase activity.
Shown is the relative β-galactosidase activity in cell extracts of *Z. bailii* ATCC 36947 cells transformed with the indicated plasmids. The β-galactosidase activity of cells transformed with $pZ_3$LacZ was set to 1 and the other activities were related to that value. Cells were grown in YPD medium (glucose 2% w/V), and samples were collected as the cultures reached an $OD^{660}$ value between 1 and 2.

**a)** Different promoters in the same plasmid. $pZ_3$: *Sc*TPI, $pZ_3$*b*T: *Zb*TPI, $pZ_3$*r*G: *ZrGAPDH.*

**b)** Different plasmid constituents. $pZ_3$: *Sc* ARS/CEN, p195: *Sc* 2μm ori sequence, pEZ-IA: *Zb* 2μm ori sequence (IR-A), pEZ-IAF: Zb 2μm ori sequence (IR-A) + FLP, $pEZ_2$: *Zb* 2μm ori sequence (IR-A) + FLP +TFC, $pEZ_2$-IB: *Zb* 2μm ori sequence (IR-A) + FLP + TFC + IR-B. The table indicates the determined plasmid stability of the respective constructs.

Figure 11: Leading of the zygocin pre-sequence to the secretion of Il-1β and comparison of different leader sequences

**a)** Western Blot analyses performed on cellular extracts (i) and on supernatants (*ii*) of *Z. bailii* and *S. cerevisiae* cells transformed with the plasmid $pZ_3$kbIL-1β (and with the corresponding empty plasmid $pZ_3$) and growing on YPD medium (glucose 2% w/V). Samples were taken at the indicated times. First lane: positive control (IL-1β, human recombinant *(E. coli*)*,* Roche cat n° 1 457 756). The blotted membranes were probed with an α-IL-1β polyclonal antibody.

Western Blot analyses performed on cellular extracts (*iii*) and on supernatants (*iv*) of *Z. bailii* and S. *cerevisiae* cells transformed with the plasmid $pZ_3$kbIL-1β (and with the corresponding empty plasmid $pZ_3$) and growing on YNB medium (glucose 5% w/V). Samples were taken at the indicated times. The blotted membranes were probed with an α-IL-1β polyclonal antibody.

**b)** Western Blot analyses performed on supernatants of *Z. bailii* cells growing on YNB medium (glucose 2% w/V) transformed with the indicated plasmids. The blotted membranes were probed with an α-IL-1β polyclonal antibody.

**Figure 12:** Glucoamylase Sta2 activity in transformed *Z. bailii* or *S. cerevisiae* cells, respectively
Determination of the *S. cerevisiae var. diastaticus* glucoamylase Sta2 activity (U/OD) in the growth medium (YNB, fructose 2% w/V) of *Z. bailii* and *S. cerevisiae* cells transformed with the plasmids $pZ_3$STA2 and $pZ_3$k1STA2 and the respective empty plasmid $pZ_3$, as a control (as indicated). In the first plasmid the protein is lead to secretion from its own leader sequence, in the second from the *K. lactis* killer toxin pre-leader sequence. Measurements were repeated more times and on independent clones, and variation levels are indicated with error bars.

Examples:

The following examples are included to demonstrate preferred embodiments of the invention. It should be appreciated by those of skill in the art that the techniques disclosed in the examples which follow represent techniques discovered by the inventors to function well in the practice of the instant invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

**Example 1:** construction of *Z. bailii* expression plasmids

The Backbone of the new vector $pZ_3$ (Fig. 1a) is the basic S. *cerevisiae* expression plasmid YX022 (R&D Systems, Inc., Wiesbaden, D).

The ARS1-CEN4 fragment was taken from Ycplac33 (ATCC 87623, Genbank accession no.: X75456 L26352,). It was cutted ClaI-blunt/SpeI and cloned into pYX022 opened DraIII-blunt/SpeI (in this way the plasmid lost completely the *HIS* gene).

The plasmid obtained was opened KpnI-blunt, and here the Kan cassette, derived from pFA6-KanMX4 (Wach et al., 1994 Yeast 10, 1793-1808) was inserted. The respective fragment was taken out cutting with SphI/SacI-blunt. This kanMX module contains the known $kan^r$ open reading-frame of the E. *coli* transposon Tn903 fused to transcriptional and translational control sequences of the *TEF* gene of the filamentous fungus *Ashbya gossypii* (e.g. NRRL Y-1056). The described hybrid module permits efficient selection of transformants resistant against geneticin (G418).

The expression cassette based on the constitutive *S. cerevisiae* TPI promoter and the respective polyA, interspaced by the multi cloning site (MCS), as indicated in the Figure derives from the original pYX022 plasmid (see supplier's information).

All the other plasmids indicated in the Figures 1 to 4 derive from $pZ_3$.

For the construction of the plasmid $pZ_3$kl (Fig. 1b), the signalling pre-sequence (16 aa) of the alpha-subunit of the K1 killer toxin of *K. lactis* (Stark M.J. et al., 1986, EMBO J. 5,1995-2002) was functionally linked to the TPI promoter of the $pZ_3$ plasmid, in order to lead the secretion of the protein of interest.

For the construction of the plasmid $pZ_3$ppα (Fig. 1c), the pre-pro-α-factor signal sequence was similarly utilised and functionally inserted. The sequence was taken from the plasmid pPICZαA (Invitrogen BV, The Netherlands)

For the construction of the plasmid $pZ_3$klIL-1β (Fig. 2a), the coding sequence for the protein already fused with the killer toxin *K. lactis* signal sequence was taken cutting XbaI/EcoRI-bluntended from the plasmid pCXJ-kanI (Fleer R, et al., 1991, Gene 107, 285-95) and sub-cloned into the plasmid $pZ_3$ EcoRI bluntended and de-phosphorylated.

For the construction of the plasmid $pZ_3$ppαGFP (Fig. 2c), the fragment containing the α-factor pre-pro leader sequence in frame with the GFP coding sequence was cutted HindIII bluntended/BamHI from the plasmid pPICAGFP1 and sub-cloned in the plasmid $pZ_3$ opened EcoRI bluntended/BamHI and de-phosphorylated. The plasmid pPICAGFP1 was constructed according to Passolunghi, S., et al. by introduction of a PCR amplified GFP sequence in frame into the plasmid pPICZαA (Invitrogen BV, The Netherlands). The PCR technique is known in the art. Exemplary reference is made to Gelfand, D. H., et al., PCR Protocols: A Guide to Methods and Applications, 1990, Academic Press and Dieffenbach, C. W. et al., PCR Primer: A Laboratory Manual, Cold Spring Harbor Laboratory Press, 1995.

For the construction of the plasmid $pZ_3$ppαIL-1β (Fig. 2b), the IL-1β was PCR amplified from the plasmid $pZ_3$klIL-1β.

The oligos for the amplification are the following:

*Primer: DrdI-IL* (SEQ ID NO.: 80)
5' AAGAGACTCCAACGTCGCGCACCTGTA 3' Tm: 63°C
*Primer: IL C-term* (SEQ ID NO.: 81)

(continued)

5' AGAGGATTAGGAAGACACAAATTGCATGGTGA 3' Tm: 61 °C

The following program was used for the amplification:

| | | |
|---|---|---|
| 94°C | 5min | |
| 94°C | 45s | 10 cycles |
| 27°C | 45s | |
| 72°C | 2min | |
| 94°C | 45s | 20 cycles |
| 50°C | 45s | |
| 72°C | 2min | |
| 72°C | 7min | |
| 4°C | ∞ | |

In this way a DrdI cutting site for sub-cloning the coding sequence of the IL-1β protein in frame with the α-factor pre-pro leader sequence was introduced. The plasmid $pZ_3pp\alpha GFP$ was opened EcoRI bluntended/BamHI. The PCR fragment was cutted DrdI bluntended/BamHI. Combination resulted in the plasmid $pZ_3pp\alpha$IL-1β.

In the plasmid $pZ_3$kbIL-1β, the coding sequence of the interleukin was functionally linked to the deduced pre-leader sequence of the *Z. bailii* killer toxin zygocin (Genebank accession no.: AF515592; Weiler F. et al., 2002, Mol Microbiol. 46, 1095-105.). Essentially oligonucleotides were synthesized corresponding to the deduced pre-leader sequence of the *Z. bailii* killer toxin zygocin (SEQ ID No.: 59) and cloned into the plasmid $pZ_3$. Subsequently, the IL-1β was PCR amplified as explicated before and cloned in-frame to the zygocin pre-sequence.

For the construction of the plasmid $pZ_3$GAA (Fig. 3a), the coding sequence of the *A. adeninivorans* α-glucoamylase was cut BamHI bluntended from the plasmid pTS32x-GAA (Bui D. M., et al., 1996, Appl. Microbiol. Biotechnol. 45, 102-6) and inserted in the plasmid $pZ_3$ opened EcoRI bluntended and de-phosphorylated. For the construction of the plasmid $pZ_3$STA2, the coding sequence of the S. *cerevisiae* var. *diastaticus* amylase (comprising its own leader sequence) was cut XbaI/AseI-blunt from the plasmid pMV35 (Vanoni M. et al., 1989, Biochim Biophys Acta. 1008, 168-76) and inserted in the plasmid $pZ_3$ opened EcoRI-blunt. For the construction of the plasmid $pZ_3$klSTA2, the coding sequence of the same amylase but functionally linked to the *K. lactis* killer toxin leader sequence was cut XhoI/AseI-blunt from the plasmid pMV57 (Venturini M. et al., 1997, Mol Microbiol. 23, 997-1007) and inserted in the plasmid $pZ_3$ opened EcoRI-blunt.

For the construction of the plasmid $pZ_3$LacZ (Fig. 3b), the coding sequence of the bacterial β-galactosidase was cutted HindIII bluntended/BamHI from the plasmid pSV-β-galactosidase (Promega, Inc.) and inserted into the plasmid $pZ_3$ opened EcoRI bluntended/BamHI and dephosphorylated.

In the plasmid $pZ_3$*b*T (Fig. 4a), the *TPI* promoter of S. *cerevisiae* was substituted with the endogenous TPI promoter from *Z. bailii.* The sequence was PCR amplified from the genomic DNA of the *Z. bailii* strain ISA 1307, and the primers were designed according to the literature (Merico A., et al., 2001, Yeast 18, 775-80). Extraction of genomic DNA was performed according to the protocol published by Hoffman, C. S., et al., 1987, Gene 57, 267-72).

The oligos for the amplification are the following:

*TPIprob5* (SEQ ID NO.: 82)
5' ATCGTATTGCTTCCATTCTTCTTTTGTTA 3' Tm: 59.6°C
*TPIprob3* (SEQ ID NO.: 83)
5' TTTGTTATTTGTTATACCGATGTAGTCTC 3' Tm: 59.6°C

The following program was used for the amplification:

| | | |
|---|---|---|
| 94°C | 5min | |
| 94°C | 45s | 25 cycles |
| 57°C | 45s | |
| 72°C | 1min 30s | |
| 72°C | 7min | |
| 4°C | ∞ | |

The PCR fragment was sub-cloned into the vector pST-BlueI (Novagen, Perfect Blunt cloning Kit cat. no. 70191-4), according to the included protocol. Therefrom, the promoter was cut SnaBI/SacI and sub-cloned into the $pZ_3$ opened AatII bluntended/SacI (so to remove the S. *cerevisiae* TPI promoter), obtaining the desired plasmid.

For the construction of the plasmid $pZ_3$*b*TLacZ (Fig. 4b), the coding sequence of the bacterial β-galactosidase was cutted HindIII/BamHI bluntended from the plasmid pSV-β-galactosidase (Promega, Inc.; Genebank accession no.: X65335) and inserted into the plasmid $pZ_3$*b*T opened NheI bluntended and dephosphorylated.

In the plasmid $pZ_3$*r*G, the TPI promoter of *S. cerevisiae* was substituted with the GAPDH promoter from *Z. rouxii.* The sequence was PCR amplified from genomic DNA of the *Z. rouxii.* strain LST11, and the primers were designed according to the literature (Ogawa Y. et al., 1990, Agric Biol Chem. 54, 2521-9). Extraction of genomic DNA was performed according to the protocol previously mentioned. (Another possible strain is *Z. rouxii* NRRL Y-229.)

The oligos for the amplification are the following:

pZrGAPDH_fwd (SEQ ID NO.: 93)
5' TGCAGAAAGCCCTAAGATGCT 3' Tm: 60.3°C
pZrGAPDH_rev (SEQ ID NO.: 94)
5' TGTCTGTGATGTACTTTTTATTTGATATG 3' Tm: 59.2°C

The following program was used for the amplification:

| | | |
|---|---|---|
| 94°C | 5min | |
| 94°C | 15s | 25 cycles |
| 57°C | 30s | |
| 72°C | 45s | |
| 72°C | 7min | |
| 4°C | ∞ | |

The obtained PCR fragment (708 bp) was sub-cloned into the vector pST-BlueI (Novagen, Perfect Blunt cloning Kit cat. no. 70191-4), according to the included protocol. Therefrom, the promoter was cut SnaBI/SacI and sub-cloned into the $pZ_3$ opened AatII bluntended/SacI (so to remove the *S. cerevisiae* TPI promoter), obtaining the desired plasmid.

For the construction of the plasmid $pZ_3$rGLacZ (Fig. 4b), the coding sequence of the bacterial β-galactosidase was cut HindIII/BamHI bluntended from the plasmid pSV-β-galactosidase (Promega, Inc.; Genebank accession no.: X65335) and inserted into the plasmid $pZ_3$rG opened XhoI bluntended and de-phosphorylated.

DNA manipulation, transformation and cultivation of *E. coli* (DH5α), were performed following standard protocols (Sambrook J., et al., Molecular Cloning: A Laboratory Manual, 2nd edn., Cold Spring Harbor Laboratory, New York, 1989). Also other basic molecular biology protocols were performed following this manual if not otherwise stated. All the restriction and modification enzymes utilised are from NEB (New England Biolabs, UK) or from Roche Diagnostics.

**Example 2:** Transformation of *Z. bailii*

Transformations of all the *Z. bailii* and the *S. cerevisiae* (NRRL Y-30320) strains were performed basically according to the LiAc/PEG/ss-DNA protocol (Agatep, R., et al., 1998, Transformation of *Saccharomyces cerevisiae* by the lithium acetate/single-stranded carrier DNA/polyethylene glycol (LiAc/ss-DNA/PEG) protocol. Technical Tips Online (http://tto.trends.com)). After the transformation, *Z. bailii* cells were recovered with an incubation of 16 hours in YP medium, comprising 2% wN of fructose as carbon source (YPF), and 1 M sorbitol, at 30°C. The cell suspension was then plated on selective YPF plates with 200 mg/l G418 (Gibco BRL, cat. 11811-031). Single clones appeared after 2-3 days at 30°C. From then on the transformants were grown either in rich or in minimal medium having glucose as carbon source and 200 mg/l G418 for maintenance of the selection. For S. *cerevisiae* cells, the procedure was the same, except for the carbon source, that remained glucose in all the steps, and for the G418 concentration, optimised for our strain to 500 mg/l.

Example 3: Expression and secretion of Interleukin 1-β in *Z. bailii*

In order to check the secretory capability of the yeast *Z. bailii* and to compare it with the well known host *S. cerevisiae,* both yeasts were transformed (according to Example 2) with the plasmid $pZ_3$kIIL- 1β (Fig. 2a). Independent transformants were shake flask cultured in minimal medium (YNB, 1.34% wN YNB from Difco Laboratories, Detroit, MI #919-15, 5% wN Glucose, complemented with Histidine, Uracil and Leucine, Fig. 5a, left panel) or in rich medium (YPD, 5% wN Glucose, 2% wN Peptone, 1% wN Yeast extract, Fig. 5a, right panel). Fig. 5a shows the cell density (OD 660nm) and the glucose consumption during the kinetics of growth. The glucose consumption was determined using a commercially available enzymatic kit from Boehringer Mannheim GmbH, Germany (Cat # 716251), according to the manufacturer's instructions. During the kinetics, samples were collected at the indicated times (see "hours" of Fig. 5b, c, d). Cells were harvested (a culture volume corresponding to $10^8$ cells) by centrifugation (10 min 10.000 rpm). 1 volume 2X Laemmli Buffer (Laemmli, U.K., 1970, Nature 227, 680-5) was added to the supernatants of said samples, they were boiled 3-5 minutes and stored at -20°C until loading or loaded directly on a polyacrylamide gel.

The cell pellets of said samples were resuspended in 5ml 20% TCA, centrifuged (10 min at 3000 rpm) and the resulting pellets were resuspended in 150μl 5% TCA. Samples were subsequently centrifuged for 10 min at 3000 rpm, and the pellet was resuspended in Laemmli Buffer (100μl). In order to neutralise the samples, 1 M Tris base was added (50μl). After 3-5 min at 99°C the samples are ready to be loaded on a polyacrylamide gel (alternatively, they can be stored at - 20°C).

Samples were loaded on standard polyacrylamide gels (SDS-PAGE, final concentration of the separating gel: 15%); after protein separation, gels were blotted (1 h, 250 mA) to nitrocellulose membranes (protran BA 85, Schleicher & Schuell). Immunodecoration: after 1h (RT) of saturation in TBS 1X (1.2 g/l Tris base; 9 g/l NaCl) + 5% NFM (non fat milk), 0.2% Tween-20, the membranes were incubated overnight at 4°C with the primary antibody against interleukin (rabbit polyclonal antibody IL-1 β(H-153) from Santa Cruz Biotechnology, Inc. cat. n° sc-7884) diluted 1:200 in TBS 1X (1.2 g/l Tris base; 9 g/l NaCl) + 5% NFM. After intensive and repeated washes in TBS + 0.2% Tween-20, the secondary antibody (antirabbit IG horseradish peroxidase-conjugated, Amersham Biosciences, UK cat n° NA934) was added (1: 10.000 in TBS 1X + 5% NFM) and left in incubation for 1h (RT). The proteins were visualised using ECL Western Blotting System (Amersham Biosciences, UK) according to the manufacturer's instructions.

The data obtained by Western Blot performed on the supernatant highlight the surprisingly good secretory capability of *Z. bailii* cells (see Fig. 5c), both in minimal and in rich medium. Remarkably, the signal corresponding to the secreted protein is significantly more intense compared to the signal obtained from *S. cerevisiae* cells, in agreement with the lower signal revealed in *Z. bailii* crude cell extracts (Fig. 5b). Moreover, the difference in the secreted levels of proteins is even more pronounced in minimal medium respect than in rich medium (for a comparison: Fig. 5c, left and right panel). These conclusions can be done either considering samples loaded rectifying the OD (Fig. 5c) or either considering equal volumes of loaded samples (Fig. 5d).

Similarly, *Z. bailii* and *S. cerevisiae* cells were transformed with the plasmid $pZ_3$ppαIL-1β. In this case the same protein (interleukin) is functionally fused with the leader sequence of the *S. cerevisiae* α-factor pheromone. As previously described, cells were shake flask cultured in rich YPD or in minimal YNB medium, samples were collected and prepared for protein SDS-PAGE separation.

The Western Blot (Fig. 6a) once more revealed the surprisingly better secretion occurring in *Z. bailii* if compared to *S. cerevisiae:* the signals obtained from the crude extracts (*i* for YPD, *iii* for YNB medium) are more intense in the latter strain, suggesting that the product is shorter retained and therefore more efficiently secreted from *Z. bailii* cells. This observation is consistent with the fact that the signals corresponding to the product secreted into the medium are more intense in *Z. bailii* samples than in *S. cerevisiae* ones (*ii* for YPD, *iv* for YNB medium; in this case a positive signal is present only in *Z. bailii* samples).

Importantly, the process of expression, secretion and accumulation of heterologous proteins in the culture medium can be obtained not only by changing the leader sequence, but also by utilising the same leader sequence but changing the heterologous protein expressed. *Z. bailii* cells were transformed with the plasmid $pZ_3pp\alpha GFP$, shake flask cultured in minimal YNB medium, samples were collected and prepared for protein SDS-PAGE separation. The Western Blot analyses performed as previously described, except for the primary antibody utilised (anti-GFP, Clontech, Inc.) and its concentration (1:500), show a band of the expected dimension that is present only in the supernatant of the *Z. bailii* cells expressing the GFP heterologous protein (Fig. 6b) and not in the control strain, transformed with the empty plasmid.

The data obtained underline the possibility to utilise *Z. bailii* as a host for the process to express different heterologous proteins and to secrete them, leading the secretion with heterologous leader sequences. Remarkably, the level of secreted proteins are higher compared with the levels obtained in S. *cerevisiae,* and the difference is even more pronounced, in chemically defined culture medium.

**Example 4:** Expression and secretion of Interleukin 1-β in a *Z. bailii* bioreactor batch cultivation with high sugar concentration.

*Z. bailii* cells transformed (according to Example 2) with the plasmid $pZ_3$klIL-1β (Fig. 2a) and previously analysed for interleukin 1-β production in shake flask culture (see Example 3), were batch cultivated in a 2 1 laboratory bioreactor (fermentor, Biolafitte & Moritz, Mod. Prelude - France) in a chemically defined medium with high glucose content (27% w/V Glucose, 4% w/V $(NH_4)_2SO_4$, 0.4% w/V $MgSO_4$ 2.4% w/V $KH_2PO_4$, vitamins according to Verduyn, C., et al., 1992, Yeast 8, 501-17, wherein the final concentration of vitamins was set to be 24 times in respect to the indicated concentrations and trace elements according to Verduyn, C., et al., 1992, Yeast 8, 501-17, wherein the final concentration of trace elements was also set to be 24 times in respect to the indicated concentrations. (Depending on the salt tolerance of the production strain it might be useful in this context to add only a partial quantity of the salts with the glucose to the initial medium and to add the rest of the salts after the bioreaction (fermentation) has proceeded a sufficient amount of time.) The pH control (value: pH 5) is performed by the addition of 2M KOH. G418 was added to a concentration of 200mg/l G418, antifoam was added as necessary). The inoculum was prepared by pre-growing the yeast in shake flask (with a headspace-to-culture volume ratio of 4) in YPD rich medium (see above), with the addition of 200mg/l G418. Cells were harvested, washed with deionised water and inoculated in the final medium at OD 1.68 in the bioreactor. Cell culture was flushed with 90 1/h of air and the dissolved oxygen concentration was maintained at 40% of air saturation, varying the stirrer speed.

Fig. 7a shows the growth kinetics (cell density, OD 660nm), together with the glucose consumption, the ethanol production and the biomass produced (dry weight g/l). The glucose consumption and the ethanol production were determined by using commercial enzymatic kits (Boehringer Mannheim GmbH, Germany Kits Cat # 716251 and 0176290, respectively), according to the manufacturer's instructions. The determination of the cellular dry weight (biomass) was performed as described before (Rodrigues, F. et al, 2001, Appl. Environ. Microbiol. 67, 2123-8). Samples were collected at the indicated times and prepared for protein SDS-PAGE separation. The Western Blot analysis (performed as described in Example 3) shows a very strong and clean signal accumulating during time corresponding to the secreted product (lanes 2 to 5), and confirms the minimal retention of heterologous protein produced within the cells (lanes 6 to 9, Fig. 7b). This example shows the surprising and advantageous characteristic of *Z. bailii* cells to be able to grow as well as express and secrete a heterologous protein even at very high sugar concentrations. Reportedly S. *cerevisiae* does not grow any more or can grow only very poorly at such high sugar concentrations (see for example Porro, D., et al., 1991, Res. Microbiol. 142, 535-9).

**Example 5:** Expression and secretion of Glucoamylase in *Z. bailii.*

*Z. bailii* cells were transformed (according to Example 2) with the plasmid $pZ_3$GAA (Fig. 3), and with the empty plasmid $pZ_3$, as a control. Independent transformants were shake flask cultured in minimal YNB medium with 2% wN Glucose as a carbon source (+0.67 % wN YNB and aa, according to the manufacturer's protocol) till mid-exp phase (also referred to as mid-log). The β-glucoamylase activity was determined as follows: after cell density determination, the cells were harvested in order to rescue the culture supernatant. 15μl/ml 3M NaAc, pH 5.2 and 20 μl/ml 1% wN Starch (Fluka 85642 - high solubility -) were added. Subsequently, the samples were mixed well and incubated at the desired temperature (this experiment: 50°C). At time zero and every following 20 min, 1 ml of the incubated medium is taken, ice-cooled for 2 min, 50μl of Lugol solution (Fluka 62650) were added, shaken quickly and read at the spectrophotometer at λ580 nm. The slope of the resulting values corresponds to the glucoamylase activity. Fig. 8 shows the glucoamylase activity of three independent clones expressing the GAA and one negative control. The enzymatic activity is expressed in mU/OD, and it is calculated considering that 1U corresponds to the variation of 1 OD in 1 min. The values reported in the graphic were subtracted of the basic activity level of *Z. bailii,* as measured in the control sample.

*Z. bailii* and *S. cerevisiae* cells were transformed (according to Example 2) with the plasmids $pZ_3$STA2 and $pZ_3$k1STA2, and with the empty plasmid $pZ_3$, as a control. Independent transformants were shake flask cultured in minimal YNB medium with 2% wN fructose as a carbon source (+0.67 % wN YNB and aa, according to the manufacturer's protocol) till mid-exp phase (also referred to as mid-log). The α-glucoamylase activity was determined according to the literature (Modena et al., 1986, Arch of Biochem. And Biophys. 248, 138-50) as follows: after cell density determination, the cells were harvested in order to rescue the culture supernatant, and an aliquot of said supernatant is used for preparing the following reaction mix:

| | |
|---|---|
| Supernatant | 100μl |
| Maltotriose 400mM | 6.3μl |
| NaAc 200mM pH 4.6 | 125μl |
| $H_2O$ | 18.7μl |
| total | 250μl |

The mix is incubated for 1 hour at 37°C under slow agitation, and after that time an aliquot of said mixture is used to evaluate the reaction. The product of maltotriose degradation is glucose, and its concentration can be determined using a commercially available enzymatic kit from Boehringer Mannheim GmbH, Germany (Cat # 716251). 1U of glucoamylase specific activity is the quantity of enzyme necessary to release 1 μmol min-1 of glucose in said condition.

**Example 6:** Expression of β-galactosidase (β-gal) in *Z. bailii*

*Z. bailii* cells were transformed (according to Example 2) with the plasmid $pZ_3$LacZ (Fig. 3b), with the plasmid $pZ_3$*b*TLacZ (Fig. 4b), with the plasmid pZ3*r*GLacZ, and with the empty plasmid $pZ_3$, as a control. Independent transformants were shake flask cultured in YPD medium (see description above) with 2% wN Glucose as a carbon source till mid-exp phase. β-galactosidase activity determination: after cell density determination, 1 ml culture is harvested into an eppendorf tube, spun for 5 minutes (to get a hard pellet), aspirated with a pipet, (not using the vacuum line!), washed in 1 ml *Z* buffer [w/o BME - betamercaptoethanol -; Z buffer: 16.1g/l $Na_2HPO_4.7H_2O$, 5.5g/l $NaH_2PO_4.H_2O$, 0.75g/l KCl, 0.246g/1 $MgSO_4.7H_2O$], repelleted, suspended in 150μl Z buffer (with BME, 27μl/10μl), 50μl chloroform are added, 20μl 0.1% SDS and vortexed vigorously for 15". 700μl of pre-warmed ONPG (o-nitrophenyl β-D-galactopyranoside, Sigma N-1127, 1 mg/ml in Z+BME) are added, and the reaction is started at 30°C (20' to 3hr), checking the time. When the suspension turns yellow the reaction is stopped by addition of 0.5 ml of 1 M $NaCO_3$; after centrifugation for 10 min at maximum speed the sample is read at the spectrophotometer at λ420.

Fig. 8b shows the β-gal activity of three independent clones expressing the β-gal under control of the *Z. bailii TPI* promoter, two independent clones expressing the β-gal under control of the *S. cerevisiae TPI* promoter and one negative control (see the legend of the figure for indications of the respective clones). The enzymatic activity is expressed as Miller Unit/OD and it is calculated according to the following formula:

$$\text{Miller Units} = \frac{A_{420} \times 1000}{A_{660} \times \text{time (min)} \times \text{Vol (ml)}}$$

As it is readily visible, the expression from the endogenous TPI promoter is much stronger (4-5 times) than from the respective promoter from *S. cerevisiae.*

A similar series of experiments was performed in order to evaluate the efficiency of the plasmids based on the sequences of the endogenous *Z. bailii* plasmid in improving the expression levels of heterologous proteins. *Z. bailii* cells were transformed (according to Example 2) with the following plasmids: $pZ_3$LacZ (Fig. 3b), p195LacZ, pEZ-IALacZ, pEZ-IAFLacZ, $pEZ_2$LacZ and $pEZ_2$-IBLacZ. Independent transformants were grown till mid-log phase and β-galactosidase activity measured, as previously described. The corresponding data are reported in Fig. 10b.

**Example 7:** Isolation of an endogenous *Z. bailii* plasmid

*Z. bailii* strains ATCC 36947 and NCYC 1427 were cultivated and their endogenous plasmid was extracted, resulting in the plasmids $pZB_1$ and $pZB_5$ (see Figs. 9 a and b). The protocol used was a modification of a protocol by Lorincz, A., 1985, BRL Focus 6, 11, and uses glass beads to break the cells. After the DNA extraction, samples were loaded on an agarose gel and the band corresponding to the plasmid was eluted (Qiagen, QIAquick Gel Extraction Kit cat n° 28704). The plasmid extracted from NCYC 1427 was cut with EcoRI and some of the fragments were sequenced. These sequences correspond to SEQ ID No.: 63, SEQ ID No.: 64, SEQ ID No.: 65, SEQ ID No.: 66, SEQ ID No.: 67, SEQ ID No.: 68, SEQ ID No.: 69 or SEQ ID No.: 70, respectively.

**Example 8:** Sequence amplification of the open reading frames and of structural sequences of the endogenous *Z. bailii* plasmids

The genomic DNA extracted from the *Z. bailii* strains ATCC 36947 and NCYC 1427 were used as a template for the amplification of the open reading frames and of structural sequences of the endogenous *Z. bailii* plasmids.

The oligos for the amplification are the following:

| | |
|---|---|
| *5FLP* (SEQ ID NO.: 84) | |
| 5'-TAGCTACTCTTCTCCAGGTGTCATTAG-3' | Tm: 63.4 |
| *3FLP* (SEQ ID NO.: 85) | |
| 5'-CCTATGTCCGAGTTTAGCGAGCTTG-3' | Tm: 64.6 |
| *5TFC*(SEQ ID NO.: 86) | |
| 5'-AGAATGAACTCAGAGTTCTCTCTTG-3' | Tm: 59.7 |
| *3TFC* (SEQ ID NO.: 87) | |
| 5'-ATTCTATTGGGTATGTCCCCTG-3' | Tm: 58.4 |
| *5TFB* (SEQ ID NO.: 88) | |
| 5'-GTTTTTAATTTTGAAGCTCACCTTTAATTG-3' | Tm: 58.6 |
| *3TFB* (SEQ ID NO.: 89) | |
| 5'-ATTATGTTCTCCAGGGAAGAGGTTAG-3' | Tm: 61.6 |
| *5IRAARS* (SEQ ID NO.: 90) | |
| 5'-AGAATCAATCATTTAGTGTGGCAGGAG-3' | Tm: 61.9 |
| *3IRAARS* (SEQ ID NO.: 91) | |
| 5'-TAAAAACTGCCCGCCATATTTCGTC-3' | Tm: 61.3 |

The following program was used for the amplification:

| | | |
|---|---|---|
| 94°C | 5min | |
| 94°C | 15s | 25 cycles |
| 58°C | 30s | |
| 72°C | 2min | |
| 72°C | 7min | |
| 4°C | ∞ | |

The amplified fragments, sub-cloned into the vector pST-Blue1 (Novagen, Perfect Blunt cloning Kit cat. no. 70191-4), were sequenced and correspond to SEQ ID No.: 71 (IR-ARS), SEQ ID No.: 72 *(FLP),* SEQ ID No.: 74 *(TFB)* and SEQ ID No.: 76 *(TFC),* respectively.

These coding sequences are used for the construction of the expression plasmid $pEZ_1$, according to Figure 9b.

**Example 9:** Construction of expression plasmids based on replication and stability sequences from the *Z. bailii* pSB2 plasmid

The backbone of the new vectors is the basic *S. cerevisiae* multicopy plasmid Yeplac 195 (Gietz and Sugino, 1988, Gene 74, 527-34) modified to the expression plasmid pBR195, as described in Branduardi (2002, Yeast 19, 1165-70).

For the construction of the plasmid p195, the plasmid pBR195 was cut AatII/ApaI-blunt in order to excise the URA marker and the $Kan^R$ cassette, excised SphI/SacI-blunt from pFA6-KanMX4 (Wach et al., 1994 Yeast 10, 1793-1808) was here inserted. From this plasmid derives the plasmid p195LacZ: the LacZ gene was sub-cloned from the plasmid $pZ_3LacZ$ cut SphI/NheI into the new plasmid p195, opened with the same enzymes.

For the construction of the plasmids pEZ-IA and pEZ-IALacZ, the plasmids p195 and p195LacZ were opened NarI/StuI-blunt, in order to remove the *S. cerevisiae* 2μm-ori. The PCR fragment corresponding to the IR-A and ARS sequence from the pSB2 (see previous example for amplification detail) was excised EcoRI-blunt from the pST-Bluel plasmid and sub-cloned into the opened vectors just described.

For the construction of the plasmid pEZ-IAFLacZ, the plasmid pEZ-IALacZ was SmaI opened, and there the fragment corresponding to the FLP and the sequence containing its promoter, derived from the pST-Blue1 plasmid opened Acc1-blunt/SnaBI, was there sub-cloned. Said sequence was PCR amplified from the genomic DNA extracted from the *Z. bailii* strains ATCC 36947.

The oligos for the amplification are the following:

pFLP (SEQ ID NO.: 95)
5'-ACGCAAGAGAGAACTCTGAGTTCAT-3' Tm: 61.3
3FLP (SEQ ID NO.: 85)
5'-CCTATGTCCGAGTTTAGCGAGCTTG-3' Tm: 64.6

The following program was used for the amplification:

| | | |
|---|---|---|
| 94°C | 5min | |
| 94°C | 15s | 29 cycles |
| 58°C | 30s | |
| 72°C | 1min 30s | |
| 72°C | 7min | |
| 4°C | ∞ | |

For the construction of the plasmids $pEZ_2$ and $pEZ_2LacZ$, the plasmids pEZ-IA and pEZ-IALacZ were opened SmaI and the PCR fragment corresponding to the sequences of FLP and TFC and the respective promoters was excided SnaBI/SalI-blunt from the pST-Bluel plasmid and sub-cloned into the opened vectors just described.

The oligos for the amplification are the following:

5FLP (SEQ ID NO.: 84)
5'-TAGCTACTCTTCTCCAGGTGTCATTAG-3' Tm: 63.4
3TFC (SEQ ID NO.: 87)
5'-ATTCTATTGGGTATGTCCCCTG-3' Tm: 58.4

The following program was used for the amplification:

| | | |
|---|---|---|
| 94°C | 5min | |
| 94°C | 15s | 25 cycles |
| 58°C | 30s | |
| 72°C | 1min 30s | |
| 72°C | 7min | |
| 4°C | ∞ | |

For resulting in the plasmid $pEZ_2$ an additional cloning step was required, in order to re-insert the polyA: the polyA was excised NaeI/NheI-blunt from the plasmid pYX022 and was sub-cloned in the transitory plasmid BamHI-blunt and dephosphorylated.

For the construction of the plasmid $pEZ_2$-IBLacZ, the plasmid pEZ2LacZ was opened SalI-blunt and de-phosphorylated, and the fragment IR-B was therein sub-cloned. That fragment was EcoRI-blunt extracted from pST-BlueI (see previous example).

**Example 10:** Plasmid stability determination

The stability of the plasmids described in the previous example was determined as follows: independent *Z. bailii* transformants bearing the different plasmids were inoculated at a cellular density of $5x10^3$ cells/ml in rich media (YPD) and in rich selective media (YPD + G418), respectively. At To of the inoculum and then after 10 and 20 generations, 500 cells from any culture were plated 3 times on selective and non-selective agar plates, and subsequently incubated at 30°C till the colonies became visible. The ratio between the mean of the colony number grown on selective medium and the mean of the colony number grown on non selective medium gives the percentage of mitotic stability.

## Claims

**1.** Process for the production of a protein comprising

a. culturing a *Zygosaccharomyces bailii* strain containing a vector comprising the DNA sequence coding for the protein functionally linked to a signalling sequence selected from the group consisting of the signalling pre-sequence of the alpha-subunit of the K1 killer toxin of *Kluyveromyces lactis* and the signal sequence of the pre-pro $\alpha$-factor of *Saccharomyces cerevisiae,* and further functionally linked to a promoter
b. expressing and secreting the protein
c. isolating the protein.

**2.** The process of claim 1, wherein the vector is an extra-chromosomal plasmid.

**3.** The process of claim 2, wherein the plasmid is derived from an endogenous episomal plasmid from a *Z. bailii* strain.

**4.** The process of claim 1, wherein the plasmid comprises sequences for replication, stabilization and/or plasmid copy number control, obtainable from *Z. bailii.*

**5.** The process of claim 3, wherein the plasmid comprises at least 35 bases of one of the sequences selected from the list of SEQ ID No.: 63, SEQ ID No.: 64, SEQ ID No.: 65, SEQ ID No.: 66, SEQ ID No.: 67, SEQ ID No.: 68, SEQ ID No.: 69, SEQ ID No.: 70 or SEQ ID No.: 71.

**6.** The process of claims 1-5, wherein the promoter is a triose-phosphate isomerase promoter, obtainable from *Saccharomyces cerevisiae* or from *Z. bailii,* preferably from *Z. bailii.*

**7.** The process of claims 1-5, wherein the promoter is a glyceraldehyde phosphate dehydrogenase promoter, obtainable from *Saccharomyces cerevisiae, Zygosaccharomyces bailii* or *Zygosaccharomyces rouxii,* preferably from *Zygosaccharomyces rouxii.*

**8.** The process of claim 1, wherein the vector is the plasmid $pZ_3kl$ as shown in Figure 1b.

**9.** The process of claim 1, wherein the vector is the plasmid $pZ_3pp\alpha$ as shown in Figure 1c.

**10.** The process of claims 1-9, wherein the DNA sequence coding for the protein is identical to that of an animal, bacterial, fungal, plant or viral sources.

**11.** The process of claims 1-10, wherein the *Z. bailii* strain is selected from the list of: ATCC 36947, ATCC 60483, NCYC 1427 or ATCC 8766.

**12.** The process of one of the preceding claims, wherein the *Z. bailii* strain has been subjected to a selection process for improved secretion.

**13.** The process of one of the preceding claims, wherein the *Z. bailii* strain is cultivated in a chemically defined medium.

**14.** The process of one of the preceding claims, further comprising isolating the protein from the culture medium.

**15.** A *Zygosaccharomyces bailii* strain, expressing and secreting a heterologous protein, wherein the strain contains a vector comprising the DNA sequence coding for the protein, functionally linked to a signalling sequence selected from the group consisting of the signalling pre-sequence of the alpha-subunit of the K1 killer toxin of *Kluyveromyces lactis* and the signal sequence of the pre-pro α-factor of *Saccharomyces cerevisiae,* and further functionally linked to a promoter.

**Patentansprüche**

**1.** Verfahren zur Herstellung eines Proteins, wobei das Verfahren die Schritte umfasst:

a. Kultivierung eines Stammes von *Zygosaccharomyces bailii,* der einen Vektor enthält, welcher die DNA-Sequenz umfasst, welche das Protein codiert, das funktionell an eine Signalsequenz gebunden ist, die ausgewählt ist aus der Gruppe Prä-Signalsequenz der Alpha-Untereinheit des K1-Killertoxins von *Kluyveromyces lactis* und Signalsequenz des Prä-Pro-α-Faktors von *Saccharomyces cerevisiae,* und ferner funktionell an einen Promotor gebunden ist,
b. Expression und Sekretion des Proteins und
c. Isolierung des Proteins.

**2.** Verfahren nach Anspruch 1, in welchem der Vektor ein extrachromosomales Plasmid ist.

**3.** Verfahren nach Anspruch 2, in welchem das Plasmid von einem endogenen episomalen Plasmid aus dem *Z. bailii-Stamm* herrührt.

**4.** Verfahren nach Anspruch 1, in welchem das Plasmid Sequenzen für die Replikation, Stabilisierung und/oder Kontrolle der Anzahl der Plasmid-Kopien umfasst, welche aus *Z. bailii* erhalten werden können.

**5.** Verfahren nach Anspruch 3, in welchem das Plasmid mindestens 35 Basen von einer der Sequenzen umfasst, die ausgewählt sind aus der Liste: SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70 oder SEQ ID NO: 71.

**6.** Verfahren nach den Ansprüchen 1 bis 5, in welchem der Promotor ein Triosephosphat-Isomerase-Promotor ist, welcher aus *Saccharomyces cerevisiae* oder aus *Z. bailii,* vorzugsweise aus *Z. bailii* erhalten werden kann.

**7.** Verfahren nach den Ansprüchen 1 bis 5, in welchem der Promotor ein Glyceraldehyphosphat-Dehydrogenase-Promotor ist, welcher aus *Saccharomyces cerevisia, Zygosaccharomyces bailii* oder *Zygosaccharomyces rouxii,* vorzugsweise aus *Zygosaccharomyces rouxii* erhalten werden kann.

**8.** Verfahren nach Anspruch 1, in welchem der Vektor das in Figur 1b gezeigte Plasmid $pZ_3kl$ ist.

**9.** Verfahren nach Anspruch 1, in welchem der Vektor das in Figur 1c gezeigte Plasmid $pZ_3pp\alpha$ ist.

**10.** Verfahren nach den Ansprüchen 1 bis 9, in welchem die das Protein codierende DNA-Sequenz identisch mit der aus Tier-, Bakterien-, Pilz-, Pflanzen- oder Virus-Quellen ist.

**11.** Verfahren nach den Ansprüchen 1 bis 10, in welchem der *Z. bailii*-Stamm ausgewählt ist aus der Liste: ATCC 36947, ATCC 60483, NCYC 1427 oder ATCC 8766.

**12.** Verfahren nach einem der vorstehenden Ansprüche, in welchem der *Z. bailii*-Stamm einem Selektionsprozess für eine verbesserte Sekretion unterzogen worden ist.

**13.** Verfahren nach einem der vorstehenden Ansprüche, in welchem der *Z. bailii*-Stamm in einem chemisch definierten Medium kultiviert wird.

**14.** Verfahren nach einem der vorstehenden Ansprüche, welches ferner die Isolierung des Proteins aus dem Kulturmedium umfasst.

**15.** *Zygosaccharomyces bailii*-Stamm, welcher ein heterologes Protein exprimiert und sekretiert, wobei der Stamm einen Vektor enthält, welcher die DNA-Sequenz umfasst, welche das Protein codiert, das funktionell an eine Signalsequenz gebunden ist, die ausgewählt ist aus der Gruppe Prä-Signalsequenz der Alpha-Untereinheit des K1-Killertoxins von *Kluyveromyces lactis* und Signalsequenz des Prä-Pro-α-Faktors von *Saccharomyces cerevisiae,* und ferner funktionell an einen Promotor gebunden ist.

## Revendications

**1.** Procédé pour la production d'une protéine comprenant :

a. la mise en culture d'une souche *Zygosaccharomyces bailii* contenant un vecteur comprenant la séquence ADN codant pour la protéine liée de façon fonctionnelle à une séquence de signalisation choisie dans le groupe consistant en la pré-séquence de signalisation de la sous-unité alpha de la toxine tueuse K1 de *Kluyveromyces lactis* et la séquence signal du facteur pré-pro α de *Saccharomyces cerevisiae,* et, de plus liée de façon fonctionnelle à un promoteur
b. l'expression et la sécrétion de la protéine
c. l'isolation de la protéine.

**2.** Procédé selon la revendication 1, dans lequel le vecteur est un plasmide extra-chromosomique.

**3.** Procédé selon la revendication 2, dans lequel le plasmide est dérivé d'un plasmide épisomique endogène d'une souche *Z. bailii.*

**4.** Procédé selon la revendication 1, dans lequel le plasmide comprend des séquences pour la réplication, la stabilisation et/ou le contrôle du nombre de copies plasmides pouvant être obtenues à partir de *Z. bailii.*

**5.** Procédé selon la revendication 3, dans lequel le plasmide comprend au moins 35 bases de l'une des séquences choisies dans la liste de SEQ ID n° 63, SEQ ID n° 64, SEQ ID n° 65, SEQ ID n° 66, SEQ ID n° 67, SEQ ID n° 68, SEQ ID n° 69, SEQ ID n° 70 ou SEQ ID n° 71.

**6.** Procédé selon les revendications 1-5, dans lequel le promoteur est un promoteur isomérase triose-phosphate, pouvant être obtenu à partir de *Saccharomyces cerevisiae* ou à partir de *Z. bailii,* de préférence à partir de *Z. bailii.*

**7.** Procédé selon les revendications 1-5, dans lequel le promoteur est un promoteur déhydrogénase glycéraldéhyde phosphate, pouvant être obtenu à partir de *Saccharomyces cerevisiae, Zygosaccharomyces bailii* ou *Zygosaccharomyces rouxii,* de préférence à partir de *Zygosaccharomyces rouxii.*

**8.** Procédé selon la revendication 1, dans lequel le vecteur est le plasmide $pZ_3kl$, comme cela est montré sur la figure 1b.

**9.** Procédé selon la revendication 1, dans lequel le vecteur est le plasmide $pZ_3pp\alpha$, tel que montré sur la figure 1c.

**10.** Procédé selon les revendications 1-9, dans lequel la séquence d'ADN codant pour la protéine est identique à celle d'un animal, d'une bactérie, de sources fongiques, végétales ou virales.

**11.** Procédé selon les revendications 1-10, dans lequel la souche *Z. bailii* est choisie à partir de la liste de : ATCC 36947, ATCC 60483, NCYC 1427 ou ATCC 8766.

**12.** Procédé selon l'une des revendications précédentes, dans lequel la souche *Z. bailii* a été soumise à un procédé de sélection pour la sécrétion améliorée.

**13.** Procédé selon l'une des revendications précédentes, dans lequel la souche *Z. bailii* est mise en culture dans un milieu défini chimiquement.

**14.** Procédé selon l'une des revendications précédentes, comprenant de plus l'isolation de la protéine du milieu de culture.

**15.** Souche *Zygosaccharomyces bailii* exprimant et sécrétant une protéine hétérologue dans laquelle
la souche contient un vecteur comprenant la séquence d'ADN codant pour la protéine, liée de façon fonctionnelle à une séquence de signalisation choisie dans le groupe consistant en la pré-séquence de signalisation de la sous-unité alpha de la toxine tueuse K1 de *Kluyveromyces lactis* et la séquence signal du facteur pré-pro α de *Saccharomyces cerevisiae* et, de plus, liée de façon fonctionnelle à un promoteur.

# Figure 1

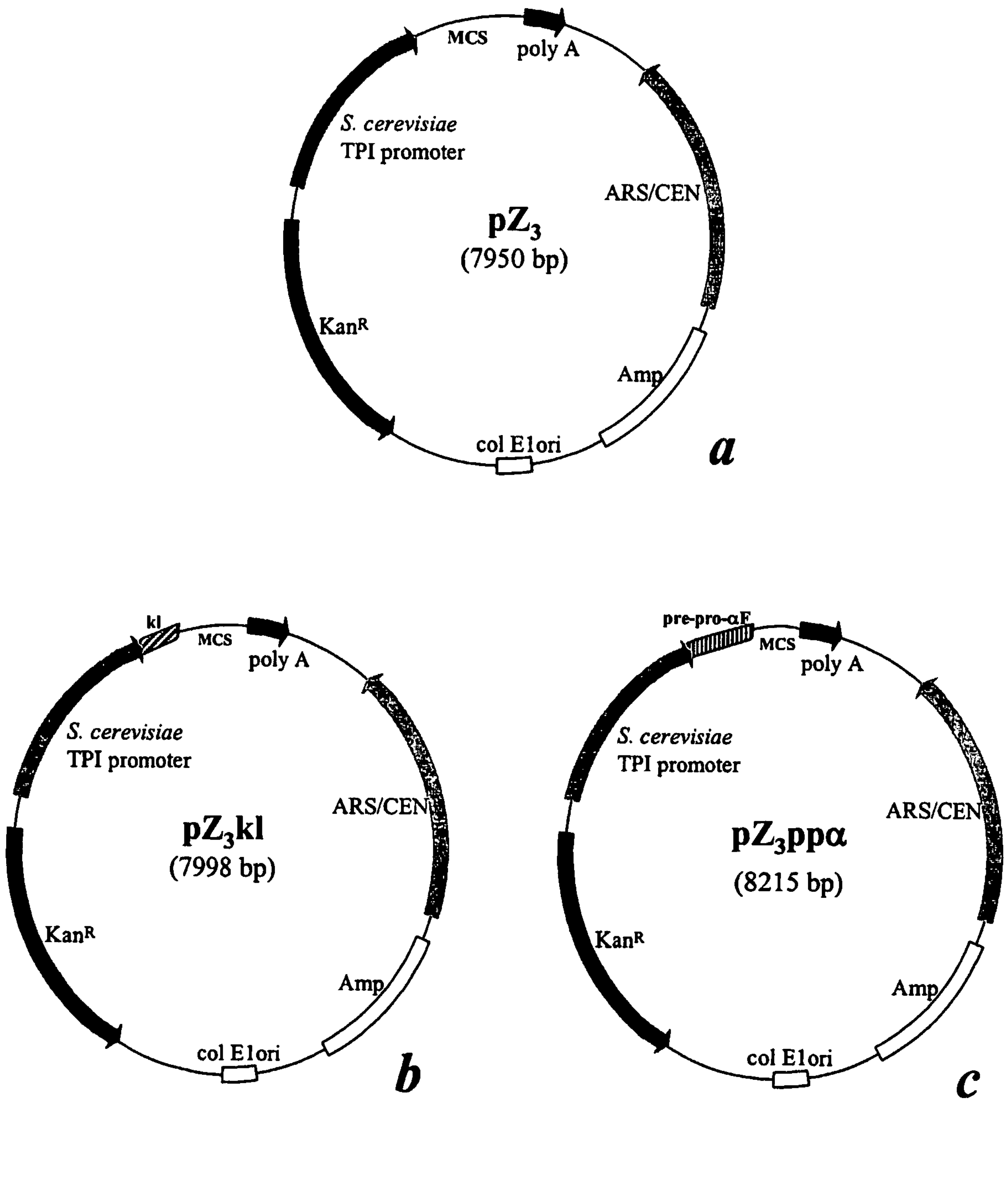
MCS
poly A
S. cerevisiae
TPI promoter
ARS/CEN
pZ3
(7950 bp)
KanR
Amp
col E1ori
a
kl
MCS
poly A
S. cerevisiae
TPI promoter
ARS/CEN
pZ3kl
(7998 bp)
KanR
Amp
col E1ori
b
pre-pro-αF
MCS
poly A
S. cerevisiae
TPI promoter
ARS/CEN
pZ3ppα
(8215 bp)
KanR
Amp
col E1ori
c

Figure 2

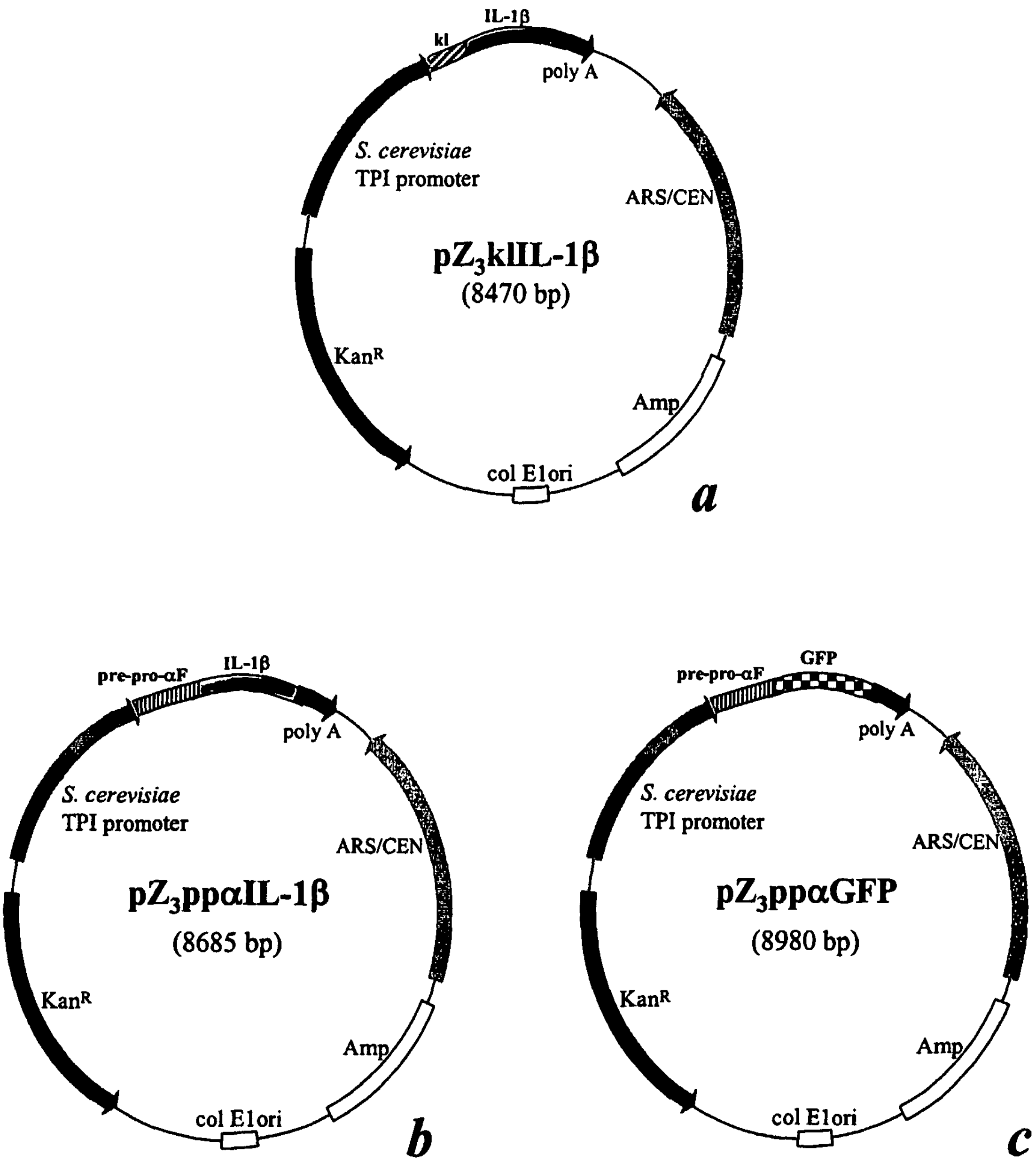
kl
IL-1β
poly A
S. cerevisiae
TPI promoter
ARS/CEN
pZ3klIL-1β
(8470 bp)
KanR
Amp
col E1ori
a
pre-pro-αF
IL-1β
poly A
S. cerevisiae
TPI promoter
ARS/CEN
pZ3ppαIL-1β
(8685 bp)
KanR
Amp
col E1ori
b
pre-pro-αF
GFP
poly A
S. cerevisiae
TPI promoter
ARS/CEN
pZ3ppαGFP
(8980 bp)
KanR
Amp
col E1ori
c

# Figure 3

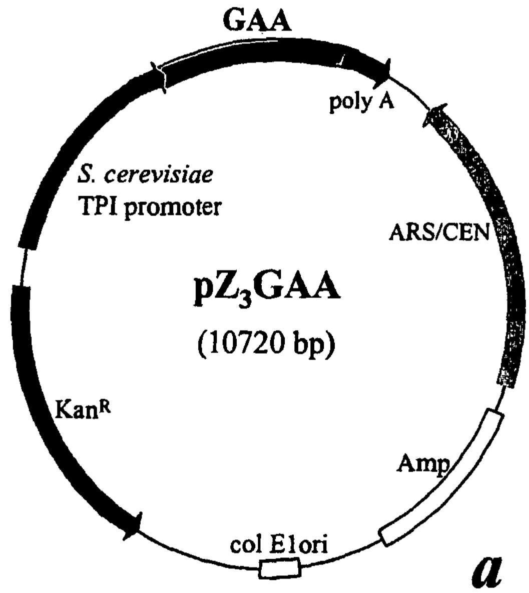
GAA
poly A
S. cerevisiae
TPI promoter
ARS/CEN
pZ3GAA
(10720 bp)
KanR
Amp
col E1ori

*a*

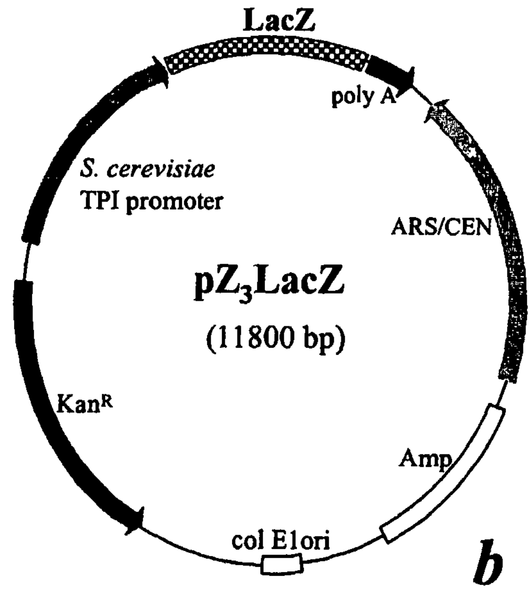
LacZ
poly A
S. cerevisiae
TPI promoter
ARS/CEN
pZ3LacZ
(11800 bp)
KanR
Amp
col E1ori

*b*

# Figure 4

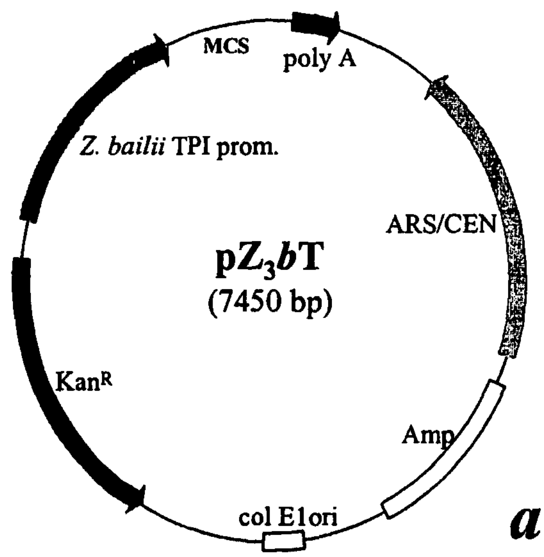
MCS
poly A
Z. bailii TPI prom.
ARS/CEN
pZ3bT
(7450 bp)
KanR
Amp
col E1ori

a

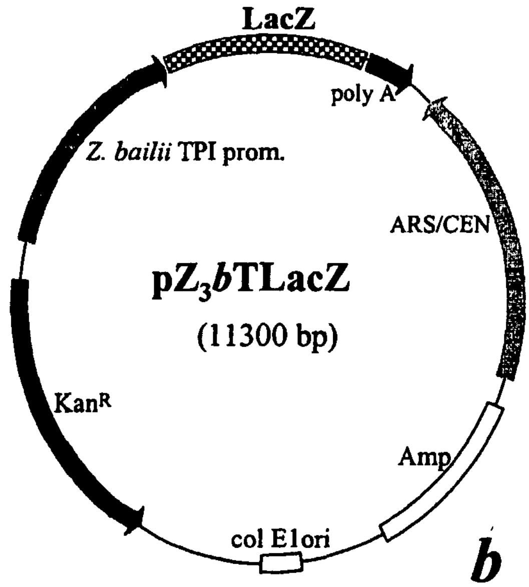
LacZ
poly A
Z. bailii TPI prom.
ARS/CEN
pZ3bTLacZ
(11300 bp)
KanR
Amp
col E1ori

b

# Figure 5

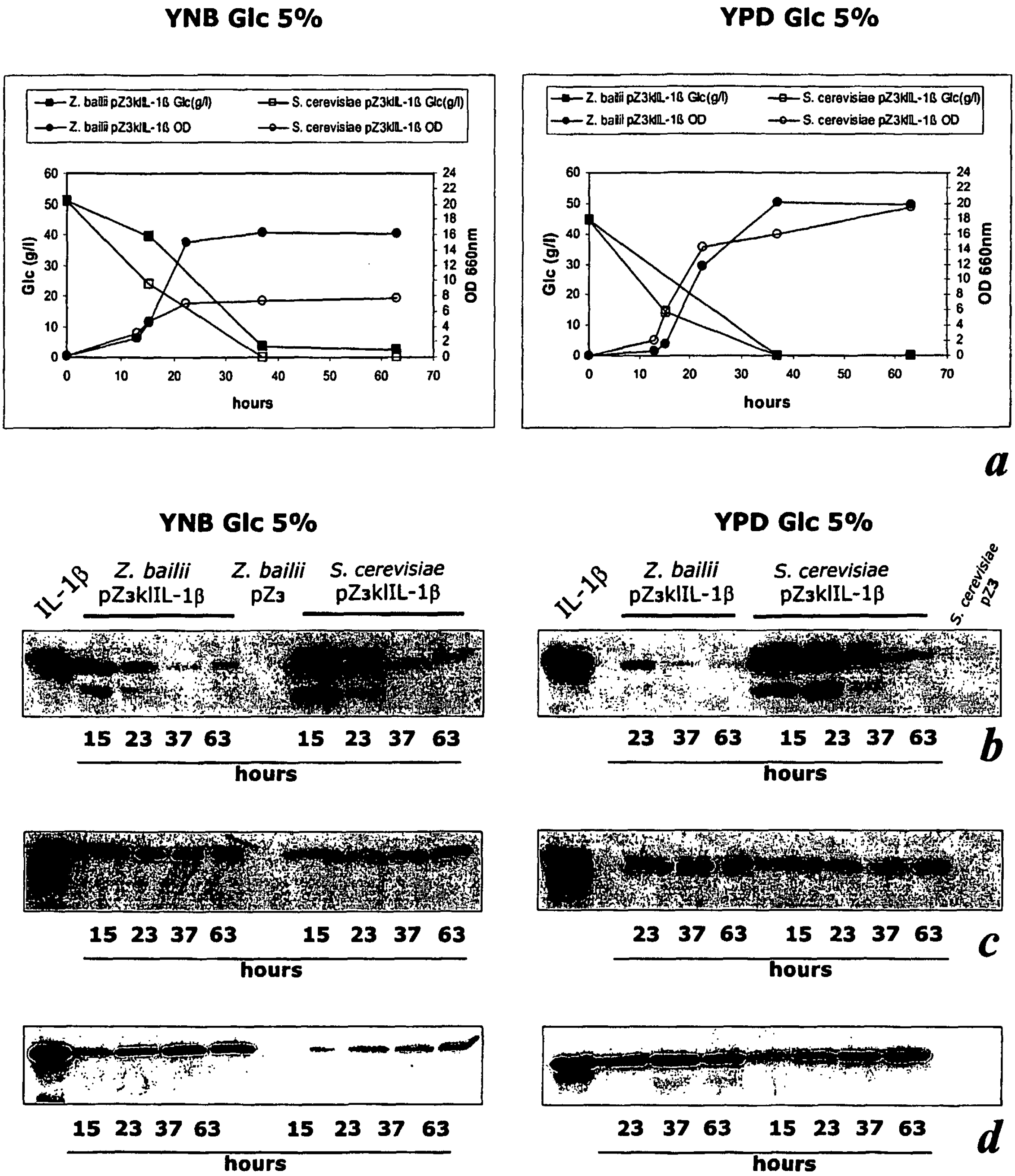
YNB Glc 5%
YPD Glc 5%
Z. bailii pZ3klIL-1ß Glc(g/l)
S. cerevisiae pZ3klIL-1ß Glc(g/l)
Z. bailii pZ3klIL-1ß OD
S. cerevisiae pZ3klIL-1ß OD
Glc (g/l)
OD 660nm
hours
a
IL-1β
Z. bailii pZ3klIL-1β
Z. bailii pZ3
S. cerevisiae pZ3klIL-1β
S. cerevisiae pZ3
15 23 37 63 15 23 37 63
23 37 63 15 23 37 63
hours
b
c
d

# Figure 6

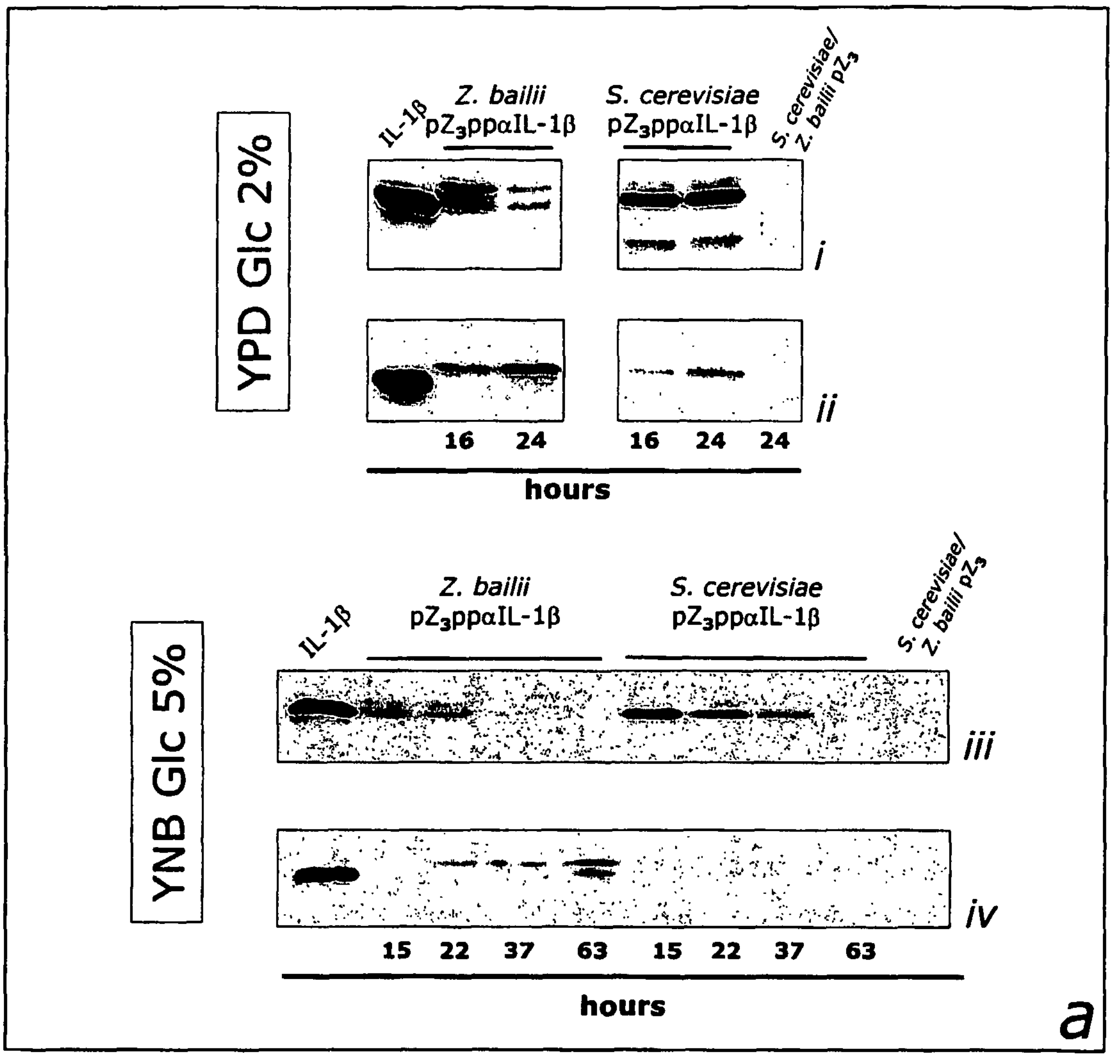
YPD Glc 2%
IL-1β
Z. bailii
pZ3ppαIL-1β
S. cerevisiae
pZ3ppαIL-1β
S. cerevisiae/
Z. bailii pZ3
i
ii
16 24 16 24 24
hours
YNB Glc 5%
iii
iv
15 22 37 63 15 22 37 63
a

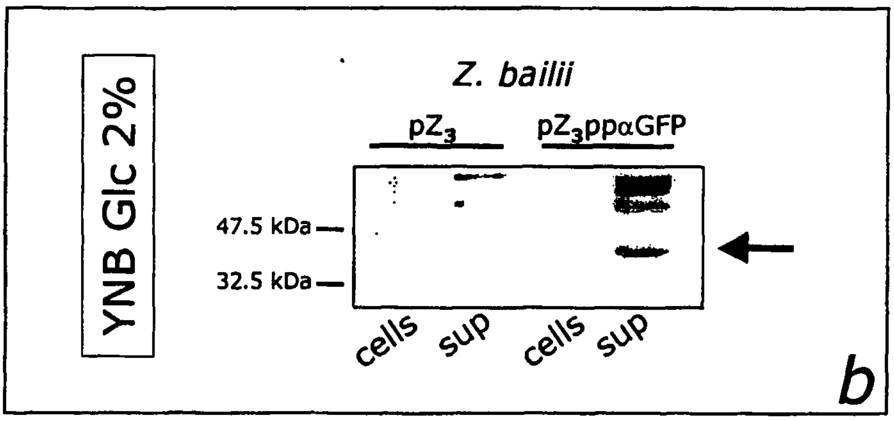
YNB Glc 2%
Z. bailii
pZ3
pZ3ppαGFP
47.5 kDa
32.5 kDa
cells sup cells sup
b

# Figure 7

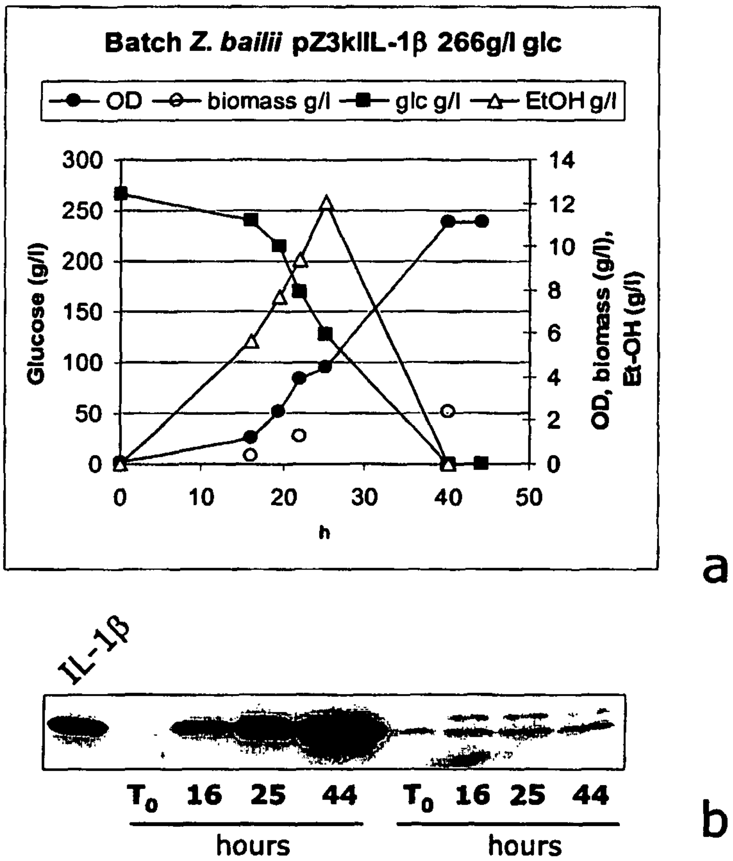
Batch Z. bailii pZ3kIIL-1β 266g/l glc
OD
biomass g/l
glc g/l
EtOH g/l
Glucose (g/l)
OD, biomass (g/l), Et-OH (g/l)
h
a
IL-1β
T0 16 25 44
hours
T0 16 25 44
hours
b

# Figure 8

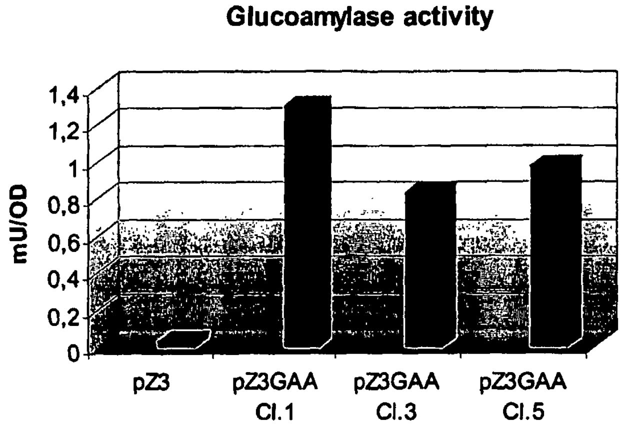
Glucoamylase activity
mU/OD
1,4
1,2
1
0,8
0,6
0,4
0,2
0
pZ3
pZ3GAA Cl.1
pZ3GAA Cl.3
pZ3GAA Cl.5

*a*

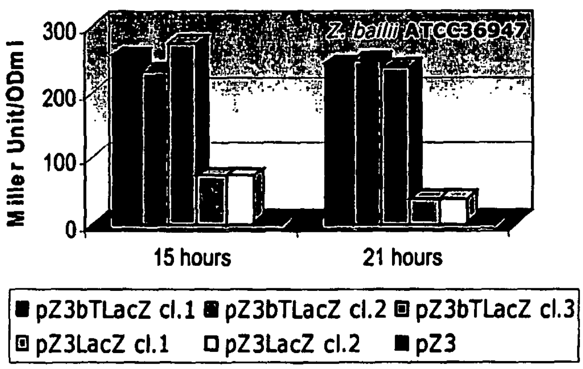
Z. bailii ATCC36947
Miller Unit/ODml
300
200
100
0
15 hours
21 hours
pZ3bTLacZ cl.1
pZ3bTLacZ cl.2
pZ3bTLacZ cl.3
pZ3LacZ cl.1
pZ3LacZ cl.2
pZ3

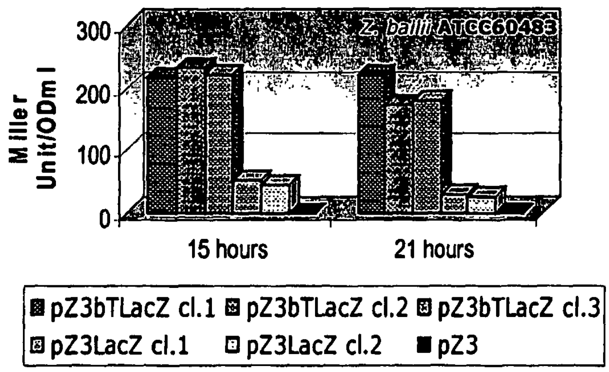
Z. bailii ATCC60483
Miller Unit/ODml
300
200
100
0
15 hours
21 hours
pZ3bTLacZ cl.1
pZ3bTLacZ cl.2
pZ3bTLacZ cl.3
pZ3LacZ cl.1
pZ3LacZ cl.2
pZ3

*b*

# Figure 9

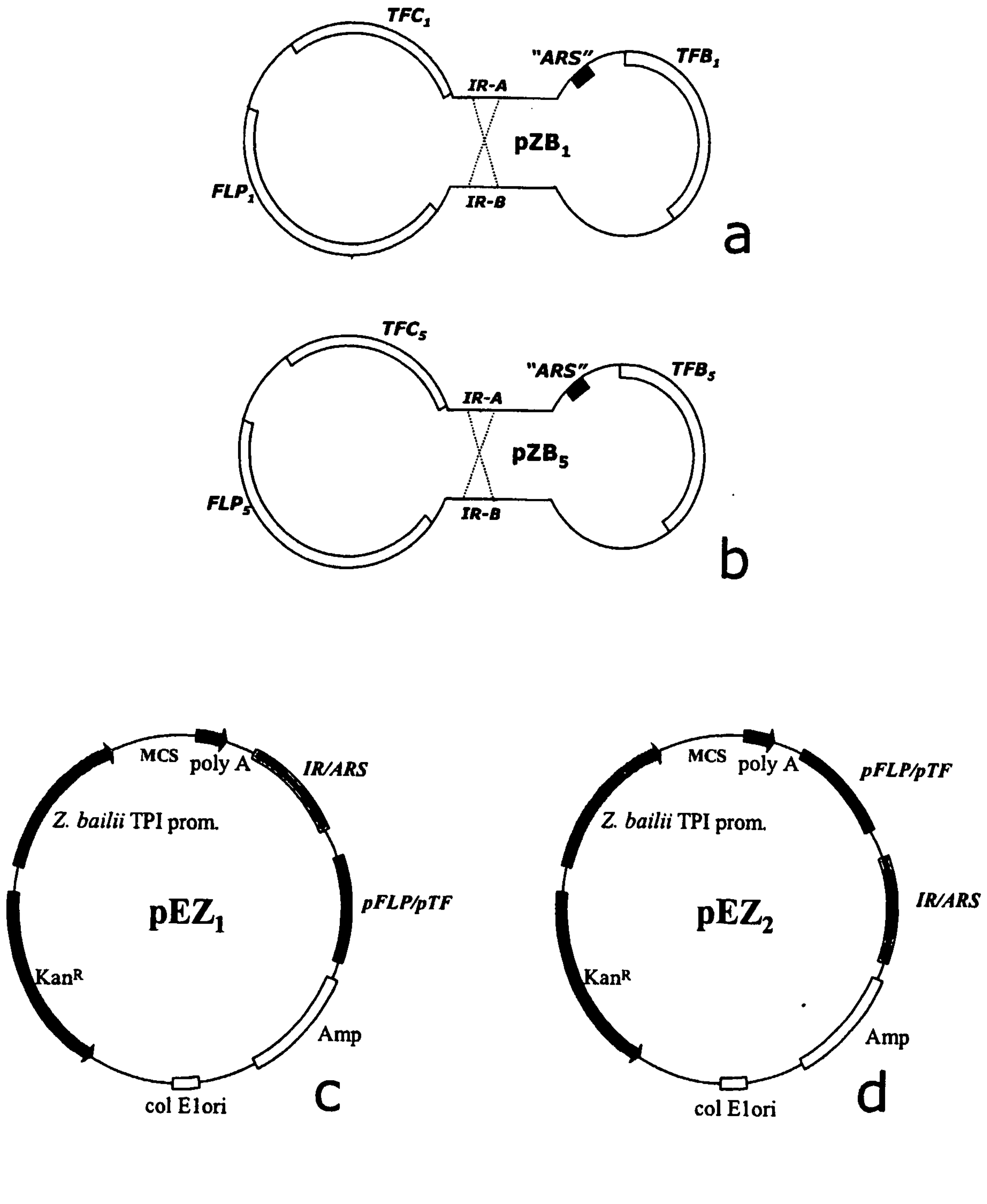
TFC1
"ARS"
TFB1
IR-A
pZB1
FLP1
IR-B
a
TFC5
"ARS"
TFB5
IR-A
pZB5
FLP5
IR-B
b
MCS
poly A
IR/ARS
Z. bailii TPI prom.
pEZ1
pFLP/pTF
KanR
Amp
col E1ori
c
MCS
poly A
pFLP/pTF
Z. bailii TPI prom.
IR/ARS
pEZ2
KanR
Amp
col E1ori
d

# Figure 10

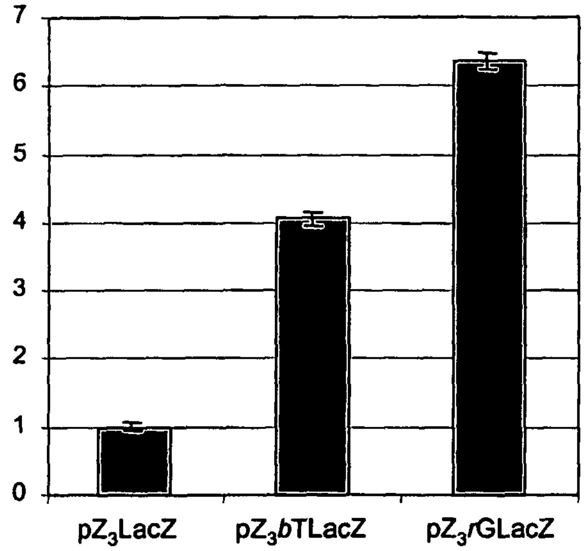
7
6
5
4
3
2
1
0
pZ3LacZ
pZ3bTLacZ
pZ3rGLacZ

*a*

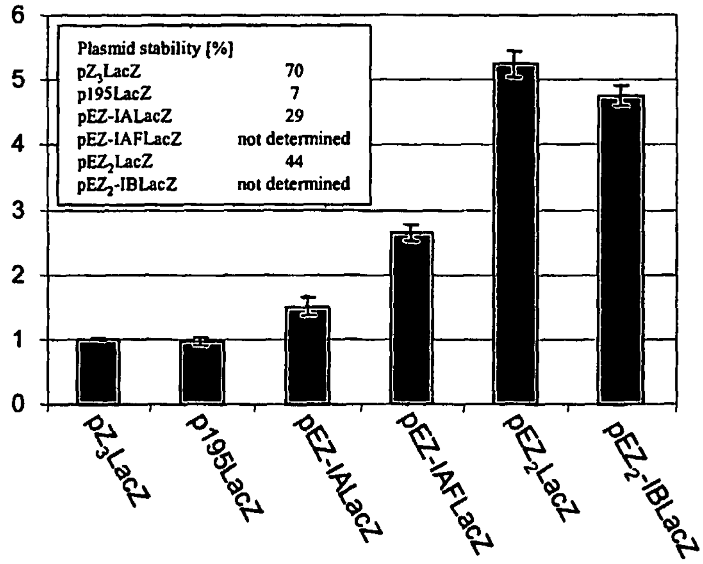
Plasmid stability [%]
pZ3LacZ 70
p195LacZ 7
pEZ-IALacZ 29
pEZ-IAFLacZ not determined
pEZ2LacZ 44
pEZ2-IBLacZ not determined
6
5
4
3
2
1
0
pZ3LacZ
p195LacZ
pEZ-IALacZ
pEZ-IAFLacZ
pEZ2LacZ
pEZ2-IBLacZ

*b*

# Figure 11

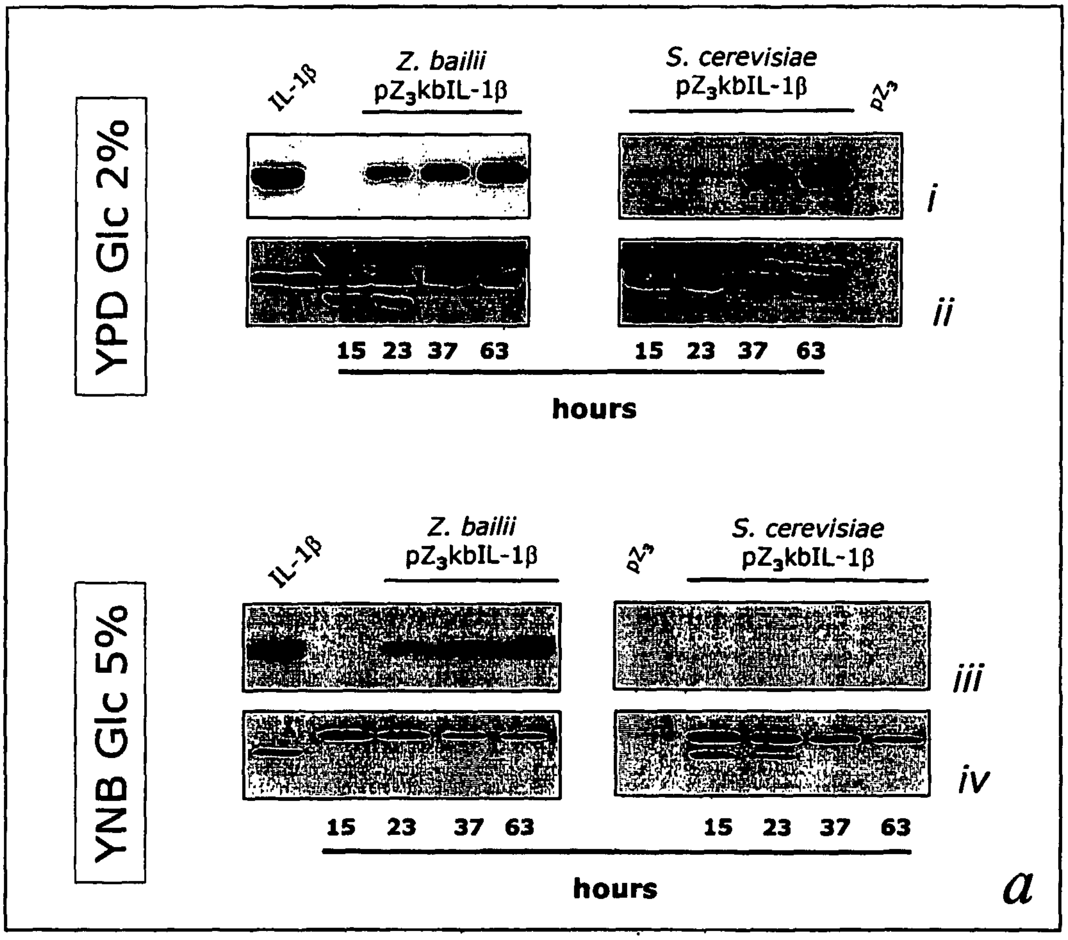
YPD Glc 2%
IL-1β
Z. bailii
pZ3kbIL-1β
S. cerevisiae
pZ3kbIL-1β
pZ3
i
ii
15 23 37 63
15 23 37 63
hours
YNB Glc 5%
IL-1β
Z. bailii
pZ3kbIL-1β
pZ3
S. cerevisiae
pZ3kbIL-1β
iii
iv
15 23 37 63
15 23 37 63
hours
a

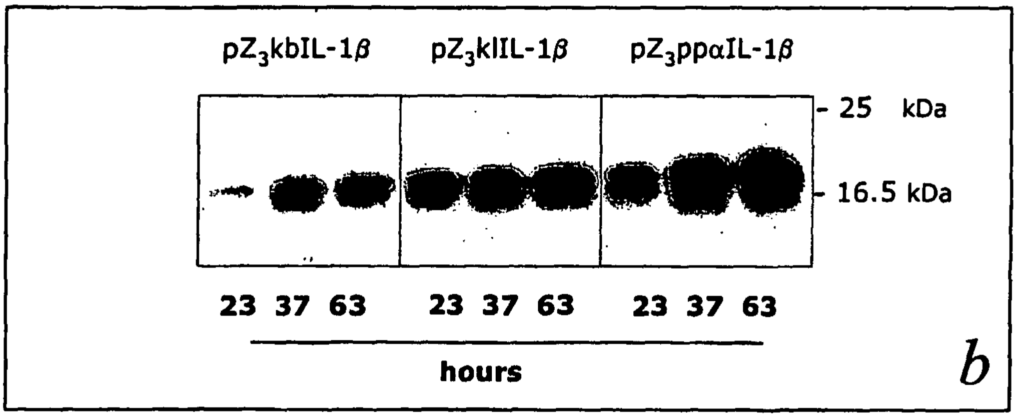
pZ3kbIL-1β
pZ3klIL-1β
pZ3ppαIL-1β
- 25 kDa
- 16.5 kDa
23 37 63
23 37 63
23 37 63
hours
b

# Figure 12

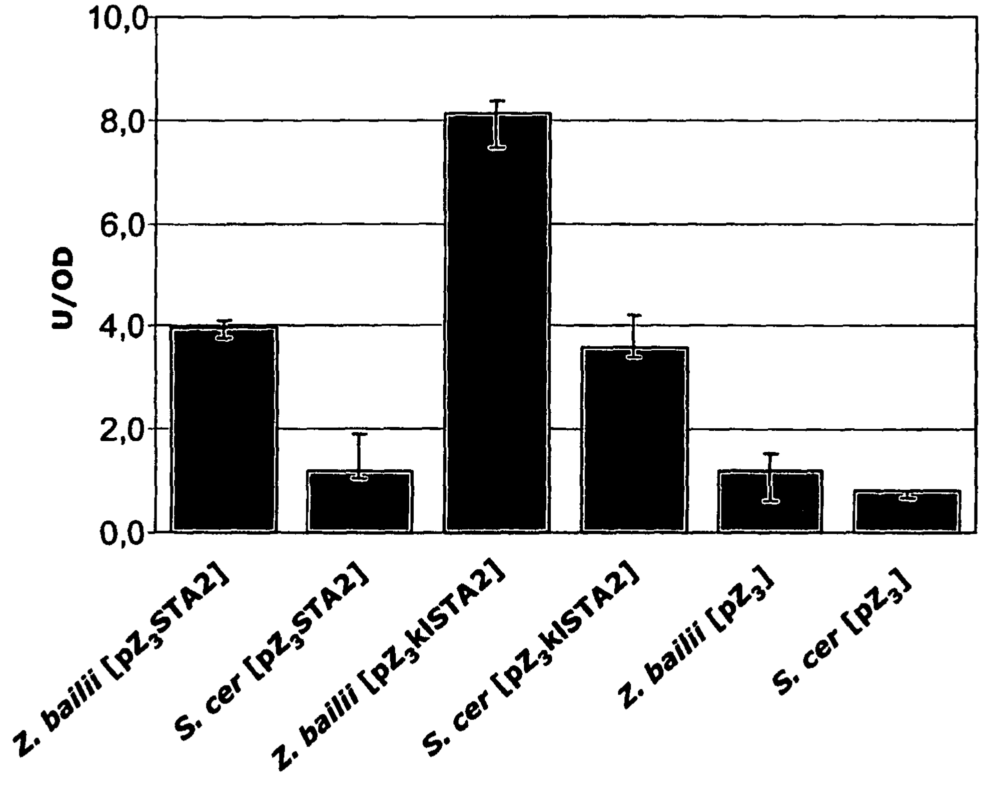
10,0
8,0
6,0
4,0
2,0
0,0
U/OD
Z. bailii [pZ3STA2]
S. cer [pZ3STA2]
Z. bailii [pZ3klSTA2]
S. cer [pZ3klSTA2]
Z. bailii [pZ3]
S. cer [pZ3]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0041477 A
- DE 10252245


### Non-patent literature cited in the description


- **HITZEMAN, R. A. et al.** *Nature,* 1981, vol. 293, 717-22
- **PORRO, D. et al.** *Res. Microbiol.,* 1991, vol. 142, 535-9
- **REISER, J. et al.** *Adv. Biochem. Eng./Biophys.,* 1990, vol. 43, 75-102
- **ROMANOS, M. A. et al.** *Yeast,* 1992, vol. 8, 423-88
- **BUCKHOLZ, R. G. et al.** *Bio/Technology,* 1991, vol. 9, 1067-72
- **FLEER, R.** *Curr. Opin. Biotechnol.,* 1992, vol. 3, 486-96
- **GELLISSEN, G. et al.** *Gene,* 1997, vol. 190, 87-97
- **MULLER, S. et al.** *Yeast,* 1998, vol. 14, 1267-83
- **JAMES, S. A. et al.** *Yeast,* 1994, vol. 10, 871-81
- **STEELS, H. et al.** *Int. J. Syst. Bacteriol.,* 1999, vol. 49, 319-27
- **KURTZMAN, C. P. et al.** *FEMS Yeast research,* 2001, vol. 1, 133-8
- **MAKDESI, A. K. et al.** *Int. J. Food Microbiol.,* 1996, vol. 33, 169-81
- **SOUSA, M. J. et al.** *Appl. Environm. Microbiol.,* 1996, vol. 62, 3152-7
- **OGAWA, Y. et al.** *Agric. Biol. Chem.,* 1990, vol. 54, 2521-9
- **FLEER, R. et al.** *Gene,* 1991, vol. 107, 285-95
- **KNIPPERS, R. et al.** Molekulare Genetik. Georg Thieme Verlag, 1990
- **STRYER, L.** Biochemie. Spektrum Akad. Verlag, 1991
- **UTATSU, I. et al.** *J. Bacteriol.,* 1987, vol. 169, 5537-45
- **BLANC H. et al.** *Mol. Gen. Genet.,* 1979, vol. 176, 335-42
- **BROACH J.R. et al.** *Cell,* 1980, vol. 21, 501-8
- **BROACH J. R. et al.** *Cell,* 1980, vol. 21, 501-8
- **BREWER B. J. et al.** *Cell,* 1987, vol. 51, 463-71
- **MCNEIL J. B. et al.** *Curr. Genet.,* 1980, vol. 2, 17-25
- **JAYARAM M. et al.** *Cell,* 1983, vol. 34, 95-104
- **VAN DEN BERG, M. et al.** *Yeast,* 1997, vol. 13, 551-9
- **BRANDUARDI, P.** *Yeast,* 2002, vol. 19, 1165-70
- **STARK M.J. et al.** *EMBO J.,* 1986, vol. 5, 1995-2002
- **VAI, M. et al.** *Appl. Environ. Microbiol.,* 2000, vol. 66, 5477-9
- **POPOLO L. et al.** *J. Bacteriol.,* 1997, vol. 180, 163-6
- **RAM A. F. J. et al.** *J. Bacteriol.,* 1998, vol. 180, 1418-24
- **KANIK-ENNULAT, C. et al.** *Mol. Cell. Biol.,* 1990, vol. 10, 898-909
- **WILLSKY. G.R. et al.** *J. Bacteriol.,* 1985, vol. 164, 611-7
- **UCCELLETTI, D. et al.** *Res. Microbiol.,* 1999, vol. 150, 5-12
- **UCCELLETTI. D. et al.** *Yeast,* 2000, vol. 16, 1161-71
- **VERDUYN, C. et al.** *Yeast,* 1992, vol. 8, 501-17
- **GIETZ, R. D. et al.** *Gene,* 1988, vol. 74, 527-34
- **WACH et al.** *Yeast,* 1994, vol. 10, 1793-808
- **AURON, E. et al.** *PNAS,* 1984, vol. 81, 7907-11
- **HEIM, R. et al.** *Curr. Biol.,* 1996, vol. 6, 178-82
- **BUI MINH, D. et al.** *Appl. Microbiol. Biotechnol.,* 1996, vol. 44, 610-9
- **WACH et al.** *Yeast,* 1994, vol. 10, 1793-1808
- **FLEER R et al.** *Gene,* 1991, vol. 107, 285-95
- **GELFAND, D. H. et al.** PCR Protocols: A Guide to Methods and Applications. Academic Press, 1990
- **DIEFFENBACH, C. W. et al.** PCR Primer: A Laboratory Manual. Cold Spring Harbor Laboratory Press, 1995
- **WEILER F. et al.** *Mol Microbiol.,* 2002, vol. 46, 1095-105

- **BUI D. M. et al.** *Appl. Microbiol. Biotechnol.,* 1996, vol. 45, 102-6
- **VANONI M. et al.** *Biochim Biophys Acta,* 1989, vol. 1008, 168-76
- **VENTURINI M. et al.** *Mol Microbiol.,* 1997, vol. 23, 997-1007
- **MERICO A. et al.** *Yeast,* 2001, vol. 18, 775-80
- **HOFFMAN, C. S. et al.** *Gene,* 1987, vol. 57, 267-72
- **OGAWA Y. et al.** *Agric Biol Chem.,* 1990, vol. 54, 2521-9
- **SAMBROOK J. et al.** Molecular Cloning: A Laboratory Manual. Cold Spring Harbor Laboratory, 1989
- **LAEMMLI, U.K.** *Nature,* 1970, vol. 227, 680-5
- **RODRIGUES, F. et al.** *Appl. Environ. Microbiol.,* 2001, vol. 67, 2123-8
- **MODENA et al.** *Arch of Biochem. And Biophys.,* 1986, vol. 248, 138-50
- **GIETZ ; SUGINO.** *Gene,* 1988, vol. 74, 527-34
- **BRANDUARDI.** *Yeast,* 2002, vol. 19, 1165-70